# EUROPEAN PATENT SPECIFICATION

(11) **EP 2 545 050 B1**
(45) Date of publication and mention of the grant of the patent: **13.08.2014**
(21) Application number: 10708084.8
(22) Date of filing: 10.03.2010
(51) Int. Cl.: C07D 471/04, A61K 31/437

(54) **COMPOUNDS FOR THE TREATMENT OF HEPATITIS C**
VERBINDUNGEN ZUR BEHANDLUNG VON HEPATITIS C
COMPOSÉS DESTINÉS AU TRAITEMENT DE L'HÉPATITE C

(43) Date of publication of application: 16.01.2013
(73) Proprietor: Bristol-Myers Squibb Company, Princeton, NJ 08543 (US)
(72) Inventor: PRACITTO, Richard, Wallingford, Connecticut 06492 (US); KADOW, John, F., Wallingford, Connecticut 06492 (US); BENDER, John, A., Wallingford, Connecticut 06492 (US); BENO, Brett, R., Wallingford, Connecticut 06492 (US); GRANT-YOUNG, Katharine, Wallingford, Connecticut 06492 (US); HAN, Ying, Wallingford, Connecticut 06492 (US); HEWAWASAM, Piyasena, Wallingford, Connecticut 06492 (US); NICKEL, Andrew, San Marino, California 91108 (US); PARCELLA, Kyle, E., Wallingford, Connecticut 06492 (US); YEUNG, Kap-Sun, Wallingford, Connecticut 06492 (US); CHUPAK, Louis, S., Wallingford, Connecticut 06492 (US)
(74) Representative: Reitstötter Kinzebach
(86) International application number: PCT/US2010/026786
(87) International publication number: WO 2011/112186

(56) References cited:
- WO-A2-2010/030538
- US-A1- 2004 162 318
- US-A1- 2006 189 606
- US-A1- 2010 184 800

## Description

### BACKGROUND OF THE INVENTION

The disclosure generally relates to the novel compounds of formula I, II, III, IV, and V, including their salts, which have activity against hepatitis C virus (HCV) and are useful in treating those infected with HCV. The disclosure also relates to compositions and methods of using these compounds.

Compounds with the above mentioned formula have been disclosed in WO2010/030538.

Hepatitis C virus (HCV) is a major human pathogen, infecting an estimated 170 million persons worldwide - roughly five times the number infected by human immunodeficiency virus type 1. A substantial fraction of these HCV infected individuals develop serious progressive liver disease, including cirrhosis and hepatocellular carcinoma (Lauer, G. M.; Walker, B. D. N. Engl. J. Med. 2001, 345, 41-52).

HCV is a positive-stranded RNA virus. Based on a comparison of the deduced amino acid sequence and the extensive similarity in the 5'-untranslated region, HCV has been classified as a separate genus in the Flaviviridae family. All members of the Flaviviridae family have enveloped virions that contain a positive stranded RNA genome encoding all known virus-specific proteins via translation of a single, uninterrupted, open reading frame.

Considerable heterogeneity is found within the nucleotide and encoded amino acid sequence throughout the HCV genome. At least six major genotypes have been characterized, and more than 50 subtypes have been described. The major genotypes of HCV differ in their distribution worldwide, and the clinical significance of the genetic heterogeneity of HCV remains elusive despite numerous studies of the possible effect of genotypes on pathogenesis and therapy.

The single strand HCV RNA genome is approximately 9500 nucleotides in length and has a single open reading frame (ORF) encoding a single large polyprotein of about 3000 amino acids. In infected cells, this polyprotein is cleaved at multiple sites by cellular and viral proteases to produce the structural and non-structural (NS) proteins. In the case of HCV, the generation of mature non-structural proteins (NS2, NS3, NS4A, NS4B, NS5A, and NS5B) is effected by two viral proteases. The first one is believed to be a metalloprotease and cleaves at the NS2-NS3 junction; the second one is a serine protease contained within the N-terminal region of NS3 (also referred to as NS3 protease) and mediates all the subsequent cleavages downstream of NS3, both in cis, at the NS3-NS4A cleavage site, and in trans, for the remaining NS4A-NS4B, NS4B-NS5A, NS5A-NS5B sites. The NS4A protein appears to serve multiple functions, acting as a cofactor for the NS3 protease and possibly assisting in the membrane localization of NS3 and other viral replicase components. The complex formation of the NS3 protein with NS4A seems necessary to the processing events, enhancing the proteolytic efficiency at all of the sites. The NS3 protein also exhibits nucleoside triphosphatase and RNA helicase activities. NS5B (also referred to as HCV polymerase) is a RNA-dependent RNA polymerase that is involved in the replication of HCV. The HCV NS5B protein is described in "Structural Analysis of the Hepatitis C Virus RNA Polymerase in Complex with Ribonucleotides (Bressanelli; S. et al., Journal of Virology 2002, 3482-3492; and Defrancesco and Rice, Clinics in Liver Disease 2003, 7, 211-242.

Currently, the most effective HCV therapy employs a combination of alpha-interferon and ribavirin, leading to sustained efficacy in 40% of patients (Poynard, T. et al. Lancet 1998, 352, 1426-1432). Recent clinical results demonstrate that pegylated alpha-interferon is superior to unmodified alpha-interferon as monotherapy (Zeuzem, S. et al. N. Engl. J. Med. 2000, 343, 1666-1672). However, even with experimental therapeutic regimens involving combinations of pegylated alpha-interferon and ribavirin, a substantial fraction of patients do not have a sustained reduction in viral load. Thus, there is a clear and important need to develop effective therapeutics for treatment of HCV infection.

HCV-796, an HCV NS5B inhibitor, showed an ability to reduce HCV RNA levels in patients. The viral RNA levels decreased transiently and then rebounded during dosing when treatment was with the compound as a single agent but levels dropped more robustly when combined with the standard of care which is a form of interferon and ribavirin. The development of this compound was suspended due to hepatic toxicity observed during exteneded dosing of the combination regimens. US patent 7,265,152 and the corresponding PCT patent application WO2004/041201 A2 describe compounds of the HCV-796 class.

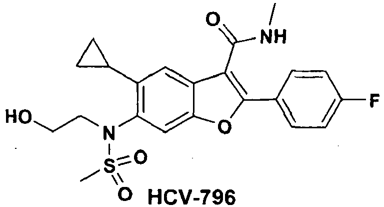

US 2006/0189606 describes indole derivatives for treating HCV infections.

The invention provides technical advantages, for example, the compounds are novel and are effective against hepatitis C. Additionally, the compounds provide advantages for pharmaceutical uses, for example, with regard to one or more of their mechanism of action, binding, inhibition efficacy, target selectivity, solubility, safety profiles, or bioavailability.

### DESCRIPTION OF THE INVENTION

The invention relates to a compound selected from the group consisting of
4-fluoro-2-(4-fluorophenyl)-5-(6-methoxy-2-methyl-5-(1-phenylcyclopropylcarbamoyl)pyridin-3-yl)-N-methylpyrazolo[1,5-a]pyridine-3-carboxamide;
4-fluoro-2-(4-fluorophenyl)-5-(6-methoxy-2-methyl-5-(1-(pyridin-2-yl)cyclopropylcarbamoyl)pyridin-3-yl)-N-methylpyrazolo[1,5-a]pyridine-3-carboxamide;
5-((6-(difluoromethoxy)-2-methyl-5-(1-phenylcyclopropylcarbamoyl)pyridin-3-yl)-4-fluoro-2-(4-fluorophenyl)-N-methylpyrazolo[1,5-a]pyridine-3-carboxamide;
5-((6-(difluoromethoxy)-2-methyl-5-(1-(pyridin-2-yl)cyclopropylcarbamoyl)pyridin-3-yl)-4-fluoro-2-(4-fluorophenyl)-N-methylpyrazolo[1,5-a]pyridine-3-carboxamide;
4-fluoro-2-(4-fluorophenyl)-N-methyl-5-(2-methyl-5-(1-(pyrimidin-2-yl) cyclopropyl carbamoyl)-6-((tetrahydro-2H-pyran-4-yl)methoxy)pyridin-3-yl)pyrazolo[1,5-a]pyridine-3-carboxamide;
4-fluoro-2-(4-fluorophenyl)-N-methyl-5-(2-methyl-5-(1-(pyridin-2-yl)cyclopropylcarbamoyl)-6-((tetrahydro-2H-pyran-4-yl)methoxy)pyridin-3-yl)pyrazolo[1,5-a]pyridine-3-carboxamide;
4-fluoro-2-(4-fluorophenyl)-5-(5-(1-(4-fluoropyridin-2-yl)cyclopropylcarbamoyl)-6-methoxy-2-methylpyridin-3-yl)-N-methylpyrazolo[1,5-a]pyridine-3-carboxamide;
4-fluoro-2-(4-fluorophenyl)-5-(4-methoxy-2-methyl-5-(1-(pyrazin-2-yl)cyclopropylcarbamoyl)phenyl)-N-methylpyrazolo[1,5-a]pyridine-3-carboxamide;
4-fluoro-2-(4-fluorophenyl)-5-(5-(1-(4-hydroxypyrimidin-2-yl)cyclopropylcarbamoyl)-4-methoxy-2-methylphenyl)-N-methylpyrazolo[1,5-a]pyridine-3-carboxamide; and
4-fluoro-2-(4-fluorophenyl)-5-(4-methoxy-2-methyl-5-(1-(pyridazin-4-yl)cyclopropylcarbamoyl)phenyl)-N-methylpyrazolo[1,5-a]pyridine-3-carboxamide;
or a pharmaceutically acceptable salt thereof.

Unless specified otherwise, these terms have the following meanings. "Alkyl" means a straight or branched alkyl group composed of 1 to 6 carbons. "Alkenyl" means a straight or branched alkyl group composed of 2 to 6 carbons with at least one double bond. "Cycloalkyl" means a monocyclic ring system composed of 3 to 7 carbons. "Hydroxyalkyl," "alkoxy" and other terms with a substituted alkyl moiety include straight and branched isomers composed of 1 to 6 carbon atoms for the alkyl moiety. "Haloalkyl" and "haloalkoxy" include all halogenated isomers from monohalo substituted alkyl to perhalo substituted alkyl. "Aryl" includes carbocyclic and heterocyclic aromatic substituents. Parenthetic and multiparenthetic terms are intended to clarify bonding relationships to those skilled in the art. For example, a term such as ((R)alkyl) means an alkyl substituent further substituted with the substituent R. Substituents which are illustrated by chemical drawing to bond at variable positions on a multiple ring system (for example a bicyclic ring system) are intended to bond to the ring where they are drawn to append. For example, substituents R¹ and R² of formula IV are intended to bond to the benzene ring of formula IV and not to the thiophene ring.

Ethylene means ethanediyl or -CH₂CH₂-; propylene means propanediyl or -CH₂CH₂CH₂-; butylene means butanediyl or -CH₂CH₂CH₂CH₂-; pentylene means pentanediyl or -CH₂CH₂CH₂CH₂CH₂-.
Dioxothiazinyl means

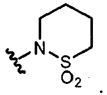

The invention includes all pharmaceutically acceptable salt forms of the compounds. Pharmaceutically acceptable salts are those in which the counter ions do not contribute significantly to the physiological activity or toxicity of the compounds and as such function as pharmacological equivalents. These salts can be made according to common organic techniques employing commercially available reagents. Some anionic salt forms include acetate, acistrate, besylate, bromide, camsylate, chloride, citrate, fumarate, glucouronate, hydrobromide, hydrochloride, hydroiodide, iodide, lactate, maleate, mesylate, nitrate, pamoate, phosphate, succinate, sulfate, tartrate, tosylate, and xinofoate. Some cationic salt forms include ammonium, aluminum, benzathine, bismuth, calcium, choline, diethylamine, diethanolamine, lithium, magnesium, meglumine, 4-phenylcyclohexylamine, piperazine, potassium, sodium, tromethamine, and zinc.

Some of the compounds of the invention possess asymmetric carbon atoms (see, for example, the structures below). The invention includes all stereoisomeric forms, including enantiomers and diastereomers as well as mixtures of stereoisomers such as racemates. Some stereoisomers can be made using methods known in the art. Stereoisomeric mixtures of the compounds and related intermediates can be separated into individual isomers according to methods commonly known in the art. The use of wedges or hashes in the depictions of molecular structures in the following schemes and tables is intended only to indicate relative stereochemistry, and should not be interpreted as implying absolute stereochemical assignments.

The invention is intended to include all isotopes of atoms occurring in the present compounds. Isotopes include those atoms having the same atomic number but different mass numbers. By way of general example and without limitation, isotopes of hydrogen include deuterium and tritium. Isotopes of carbon include ¹³C and ¹⁴C. Isotopically-labeled compounds of the invention can generally be prepared by conventional techniques known to those skilled in the art or by processes analogous to those described herein, using an appropriate isotopically-labeled reagent in place of the non-labeled reagent otherwise employed. Such compounds may have a variety of potential uses, for example as standards and reagents in determining biological activity. In the case of stable isotopes, such compounds may have the potential to favorably modify biological, pharmacological, or pharmacokinetic properties.

### Synthetic Methods

The compounds may be made by methods known in the art including those described below and including variations within the skill of the art. Some reagents and intermediates are known in the art. Other reagents and intermediates can be made by methods known in the art using readily available materials. The variables (e.g. numbered "R" substituents) used to describe the synthesis of the compounds are intended only to illustrate how to make the compounds and are not to be confused with variables used in the claims or in other sections of the specification. The following methods are for illustrative purposes and are not intended to limit the scope of the invention.

Abbreviations used in the schemes generally follow conventions used in the art. Chemical abbreviations used in the specification and examples are defined as follows: "NaHMDS" for sodium bis(trimethylsilyl)amide; "DMF" for N,N-dimethylformamide; "MeOH" for methanol; "NBS" for N-bromosuccinimide; "Ar" for aryl; "TFA" for trifluoroacetic acid; "LAH" for lithium aluminum hydride; "BOC", "DMSO" for dimethylsulfoxide; "h" for hours; "rt" for room temperature or retention time (context will dictate); "min" for minutes; "EtOAc" for ethyl acetate; "THF" for tetrahydrofuran; "EDTA" for ethylenediaminetetraacetic acid; "Et₂O" for diethyl ether; "DMAP" for 4-dimethylaminopyridine; "DCE" for 1,2-dichloroethane; "ACN" for acetonitrile; "DME" for 1,2-dimethoxyethane; "HOBt" for 1-hydroxybenzotriazole hydrate; "DIEA" for diisopropylethylamine, "Nf" for CF₃(CF₂)₃SO₂-; and "TMOF" for trimethylorthoformate.

As shown in Scheme 1, some compounds described may be prepared by coupling an aryl triflate or halide to a substituted phenyl boronic acid that in some examples contains a carboxylic acid or carboxylic acid ester. Other coupling partner, techniques and conditions are known in the art as are other carbon-carbon bond forming reactions. Acids and esters may be converted to amides by methods known in the art.

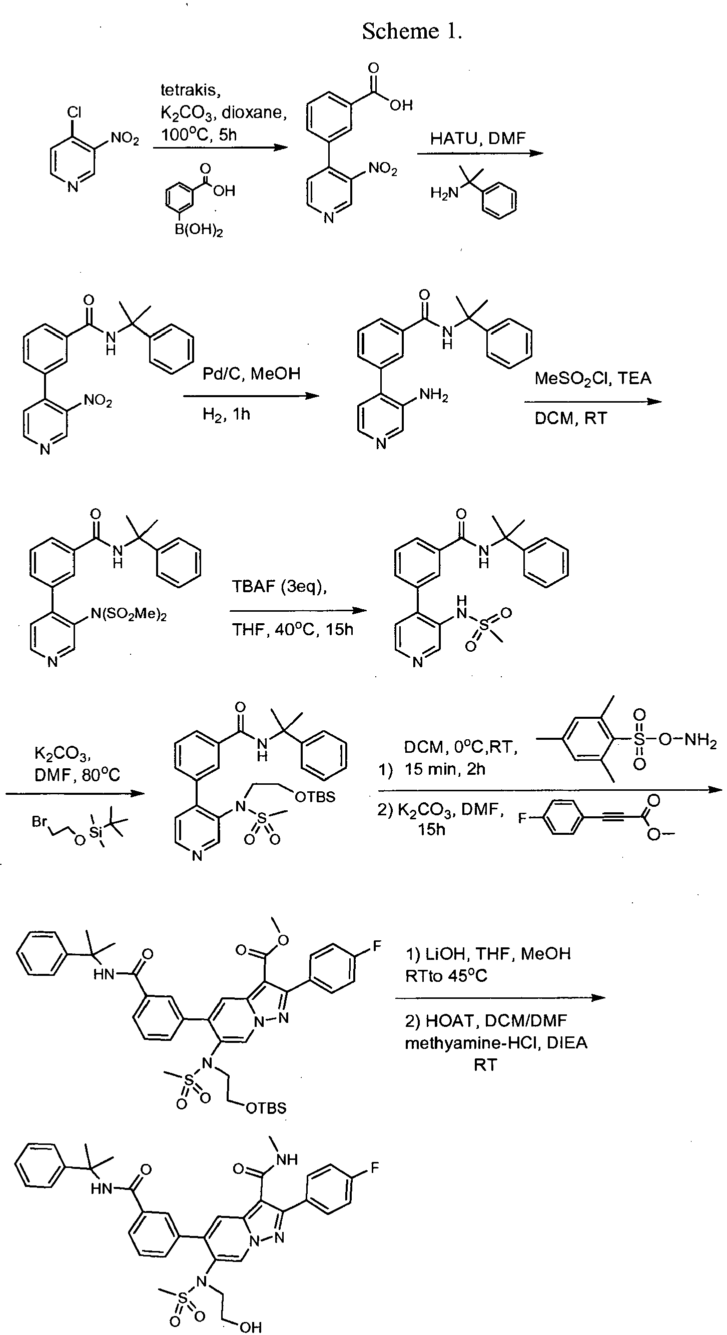

Bis-sulfonamides may be selectively hydrolyzed to a monosulfonamide. The monosulfonamide may be alkylated again with either a simple or functionalized alkyl.

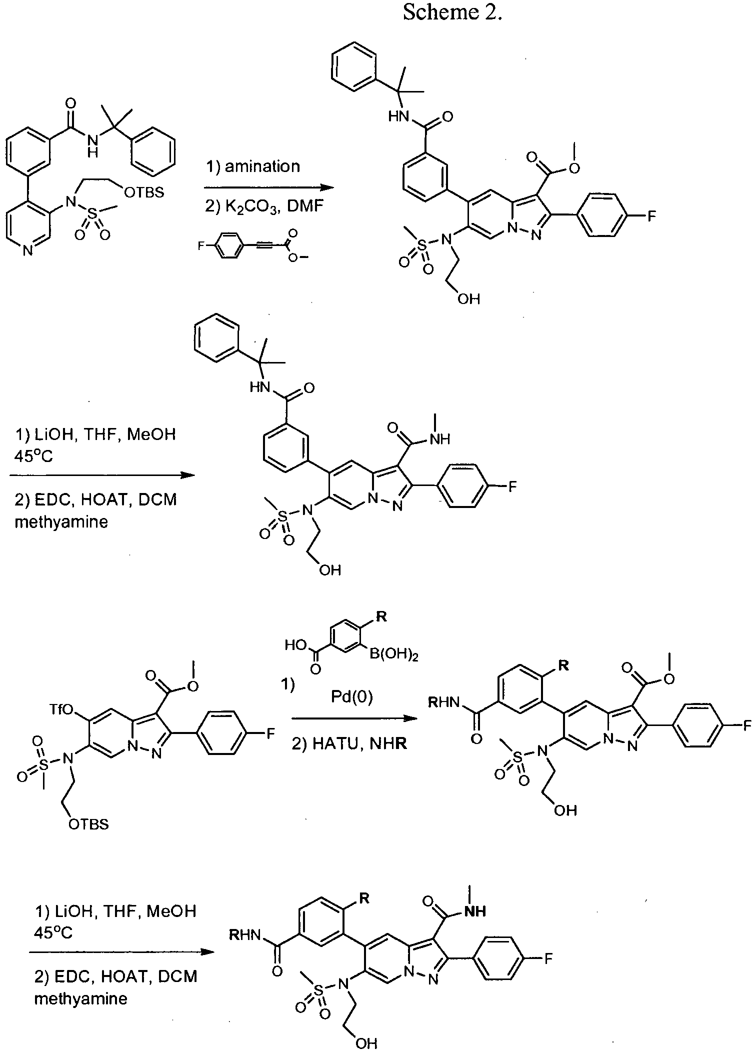

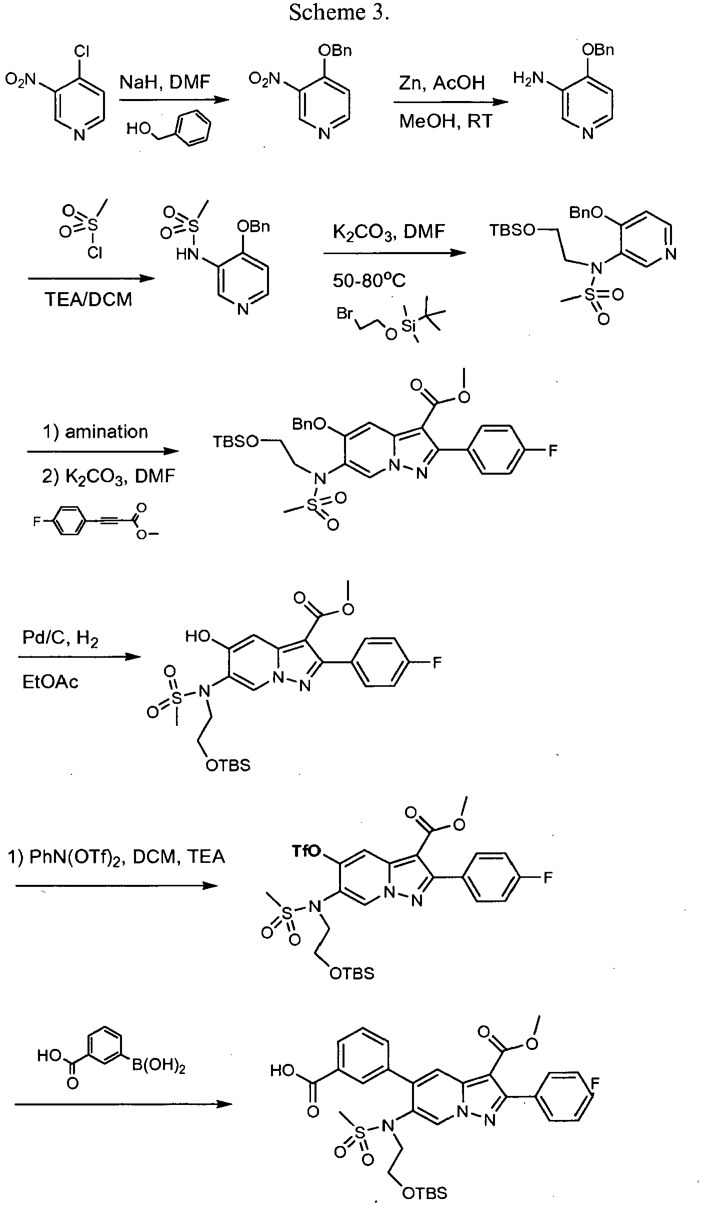

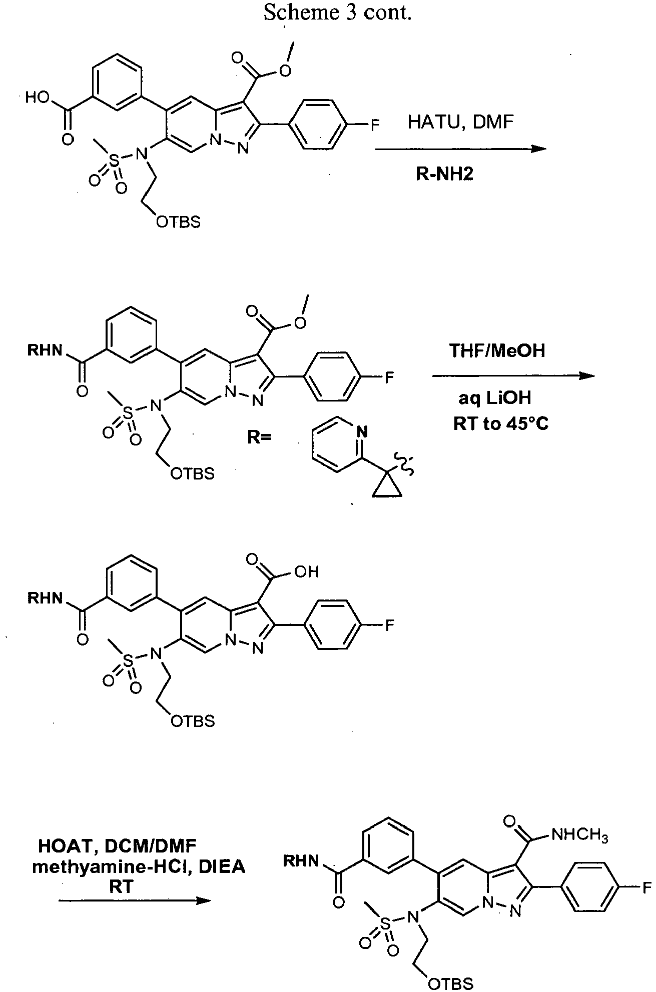

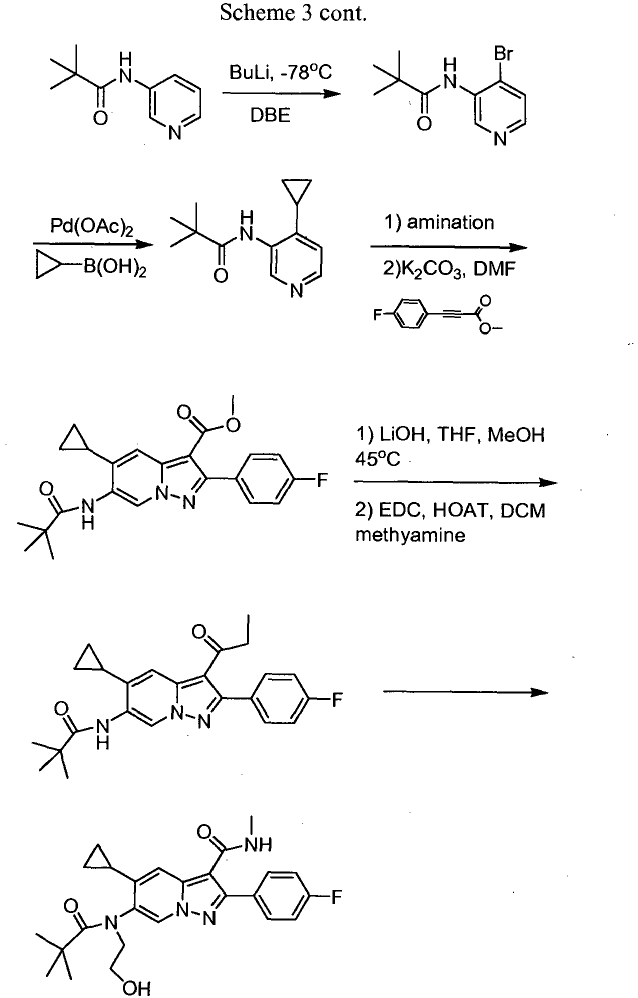

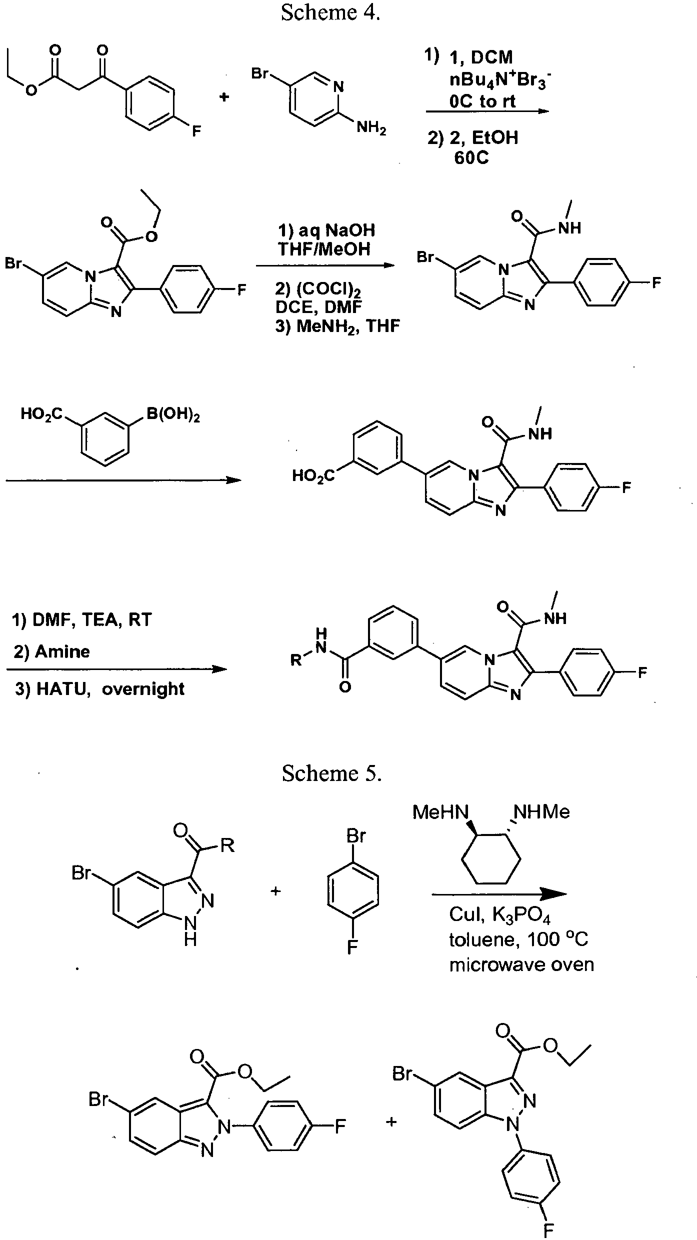

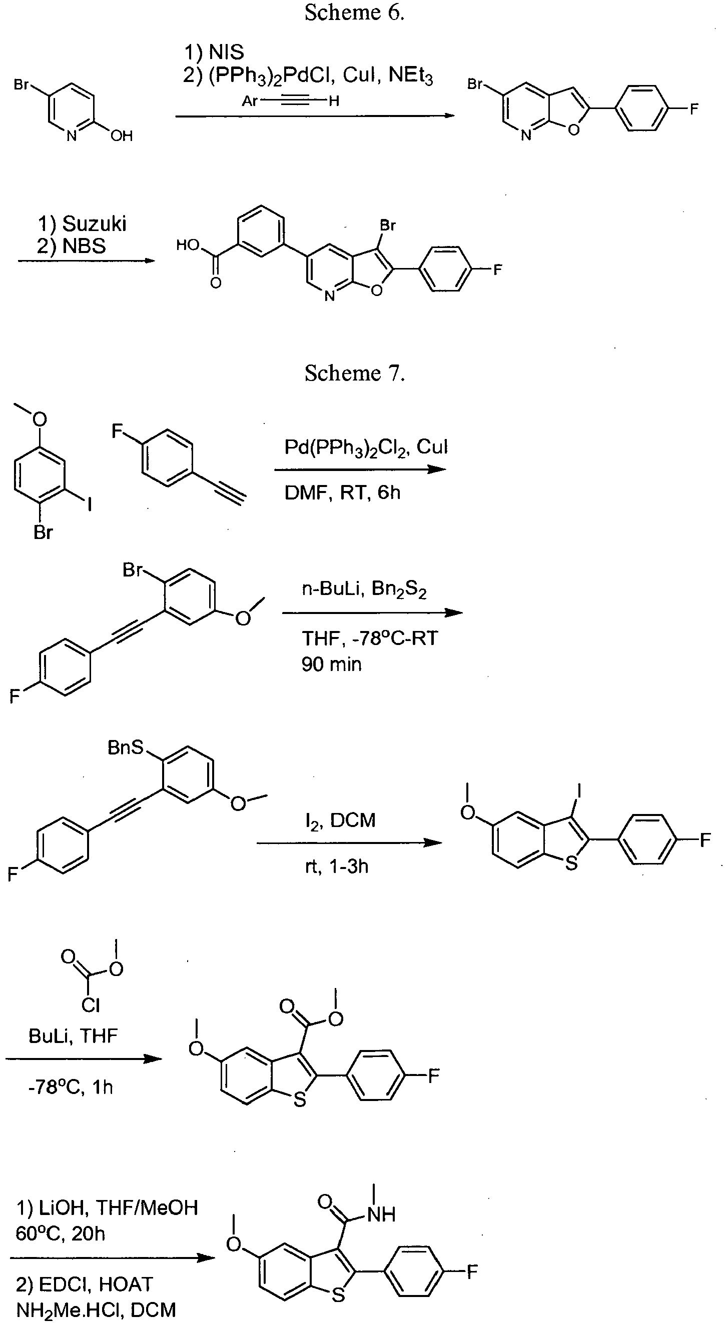

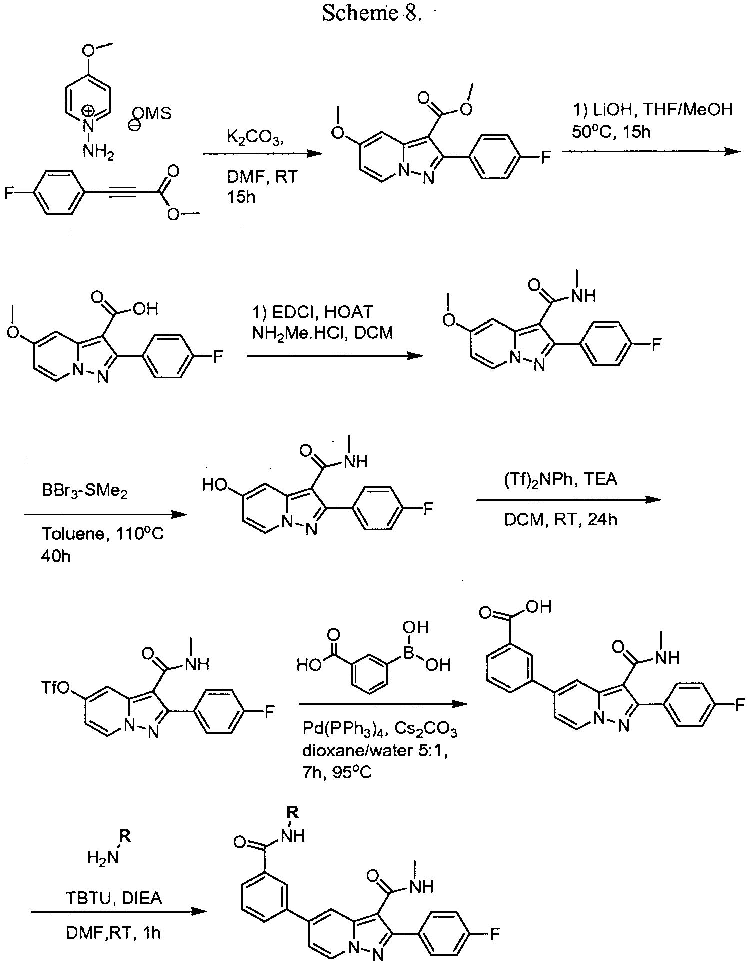

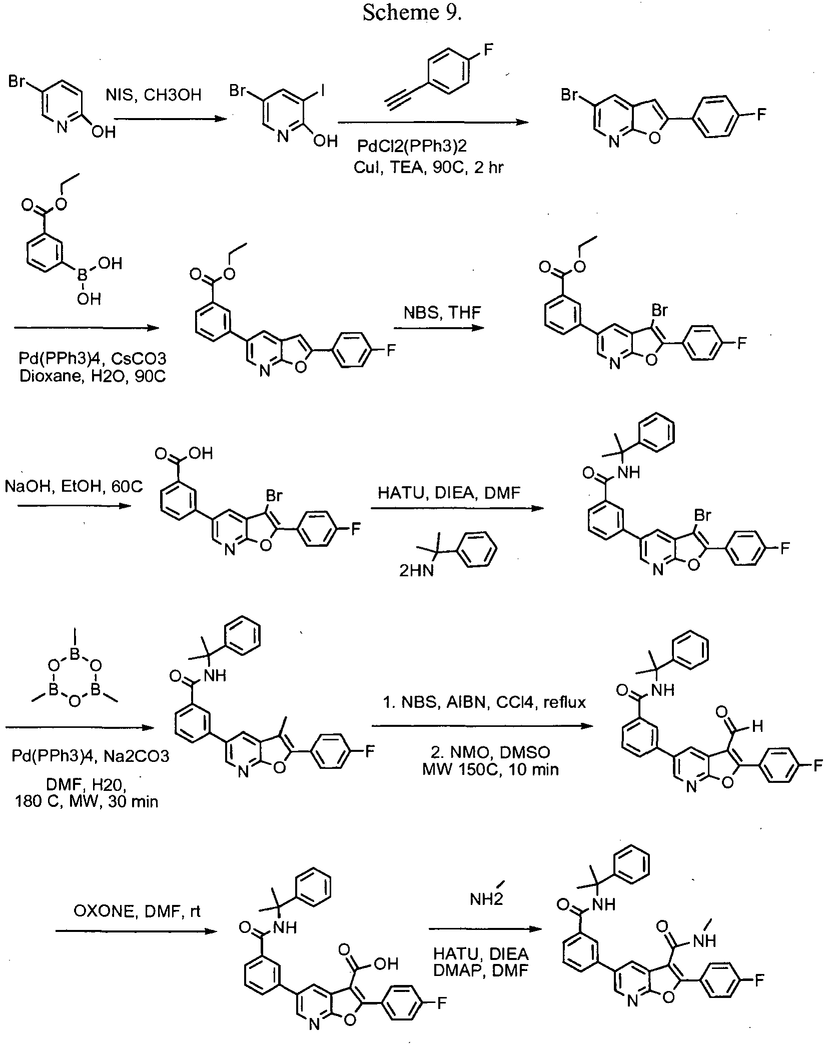

### Biological Methods

The compounds demonstrated activity against HCV NS5B as determined in the following HCV RdRp assays.

*HCV NS5B RdRp cloning, expression, and purification.* The cDNA encoding the NS5B protein of HCV, genotype 1b, was cloned into the pET21a expression vector. The protein was expressed with an 18 amino acid C-terminal truncation to enhance the solubility. The E. coli competent cell line BL21(DE3) was used for expression of the protein. Cultures were grown at 37 °C for ∼ 4 hours until the cultures reached an optical density of 2.0 at 600 nm. The cultures were cooled to 20 °C and induced with 1 mM IPTG. Fresh ampicillin was added to a final concentration of 50 µg/ml and the cells were grown overnight at 20 °C.

Cell pellets (3L) were lysed for purification to yield 15-24 mgs of purified NS5B. The lysis buffer consisted of 20 mM Tris-HCl, pH 7.4, 500 mM NaCl, 0.5% triton X-100, 1 mM DTT, 1mM EDTA, 20% glycerol, 0.5 mg/ml lysozyme, 10 mM MgCl2, 15 ug/ml deoxyribonuclease I, and Complete TM protease inhibitor tablets (Roche). After addition of the lysis buffer, frozen cell pellets were resuspended using a tissue homogenizer. To reduce the viscosity of the sample, aliquots of the lysate were sonicated on ice using a microtip attached to a Branson sonicator. The sonicated lysate was centrifuged at 100,000 x g for 1hr at 4 °C and filtered through a 0.2 µm filter unit (Coming).

The protein was purified using three sequential chromatography steps: Heparin sepharose CL-6B, polyU sepharose 4B, and Hitrap SP sepharose (Pharmacia). The chromatography buffers were identical to the lysis buffer but contained no lysozyme, deoxyribonuclease I, MgCl2 or protease inhibitor and the NaCl concentration of the buffer was adjusted according to the requirements for charging the protein onto the column. Each column was eluted with a NaCl gradient which varied in length from 5-50 column volumes depending on the column type. After the final chromatography step, the resulting purity of the enzyme is >90% based on SDS-PAGE analysis. The enzyme was aliquoted and stored at -80 °C.

*Standard HCV NS5B RdRp enzyme assay.* HCV RdRp genotype 1b assays were run in a final volume of 60 µl in 96 well plates (Costar 3912). The assay buffer is composed of 20 mM Hepes, pH 7.5, 2.5 mM KCl, 2.5 mM MgCl2, 1 mM DTT, 1.6 U RNAse inhibitor (Promega N2515), 0.1 mg/ml BSA (Promega R3961), and 2 % glycerol. All compounds were serially diluted (3-fold) in DMSO and diluted further in water such that the final concentration of DMSO in the assay was 2%. HCV RdRp genotype 1b enzyme was used at a final concentration of 28 nM. A polyA template was used at 6 nM, and a biotinylated oligo-dT12 primer was used at 180 nM final concentration. Template was obtained commercially (Amersham 27-4110). Biotinylated primer was prepared by Sigma Genosys. 3H-UTP was used at 0.6 µCi (0.29 µM total UTP). Reactions were initiated by the addition of enzyme, incubated at 30 °C for 60 min, and stopped by adding 25 µl of 50 mM EDTA containing SPA beads (4 µg/µl, Amersham RPNQ 0007). Plates were read on a Packard Top Count NXT after >1hr incubation at room temperature.

*Modified HCV NS5B RdRp enzyme assay.* A modified enzyme assay was performed essentially as described for the standard enzyme assay except for the following: The biotinylated oligo dT12 primer was precaptured on streptavidin-coated SPA beads by mixing primer and beads in assay buffer and incubating at room temperature for one hour. Unbound primer was removed after centrifugation. The primer-bound beads were resuspended in 20 mM Hepes buffer, pH 7.5 and used in the assay at final concentrations of 20 nM primer and 0.67 µg/µl beads. Order of addition in the assay: enzyme (14 nM) was added to diluted compound followed by the addition of a mixture of template (0.2 nM), 3H-UTP (0.6 µCi, 0.29 µM), and primer-bound beads, to initiate the reaction; concentrations given are final. Reactions were allowed to proceed for 4 hours at 30° C.

IC₅₀ values for compounds were determined using seven different [I]. IC₅₀ values were calculated from the inhibition using the formula y = A+((B-A)/(1+((C/x)^D))).

*FRET Assay Preparation.* To perform the HCV FRET screening assay, 96-well cell culture plates were used. The FRET peptide (Anaspec, Inc.) (Taliani et al., Anal. Biochem. 1996, 240, 60-67) contains a fluorescence donor, EDANS, near one end of the peptide and an acceptor, DABCYL, near the other end. The fluorescence of the peptide is quenched by intermolecular resonance energy transfer (RET) between the donor and the acceptor, but as the NS3 protease cleaves the peptide the products are released from RET quenching and the fluorescence of the donor becomes apparent. The assay reagent was made as follows: 5X cell Luciferase cell culture lysis reagent from Promega (#E153A) diluted to 1X with dH₂O, NaCl added to 150 mM final, the FRET peptide diluted to 20 µM final from a 2 mM stock.

To prepare plates, HCV replicon cells, with or without a Renilla luciferase reporter gene, were trypsinized and placed into each well of a 96-well plate with titrated test compounds added in columns 3 through 12; columns 1 and 2 contained a control compound (HCV protease inhibitor), and the bottom row contained cells without compound. The plates were then placed in a CO₂ incubator at 37 °C.

*Assays.* Subsequent to addition of the test compounds described above (FRET Assay Preparation), at various times the plate was removed and Alamar blue solution (Trek Diagnostics, #00-100) was added per well as a measure of cellular toxicity. After reading in a Cytoflour 4000 instrument (PE Biosystems), plates were rinsed with PBS and then used for FRET assay by the addition of 30 ul of the FRET peptide assay reagent described above (FRET Assay Preparation) per well. The plate was then placed into the Cytoflour 4000 instrument which had been set to 340 excite/490 emission, automatic mode for 20 cycles and the plate read in a kinetic mode. Typically, the signal to noise using an endpoint analysis after the reads was at least three-fold. Alternatively, after Alamar blue reading, plates were rinsed with PBS, 50 ul of DMEM (high glucose) without phenol red was added and plates were then used for luciferase assay using the Promega Dual-Glo Luciferase Assay System.

Compound analysis was determined by quantification of the relative HCV replicon inhibition and the relative cytotoxicity values. To calculate cytoxicity values, the average Alamar Blue fluorescence signals from the control wells were set as 100% non-toxic. The individual signals in each of the compound test wells were then divided by the average control signal and multiplied by 100% to determine percent cytotoxicity. To calculate the HCV replicon inhibition values, an average background value was obtained from the two wells containing the highest amount of HCV protease inhibitor at the end of the assay period. These numbers were similar to those obtained from naive Huh-7 cells.

The background numbers were then subtracted from the average signal obtained from the control wells and this number was used as 100% activity. The individual signals in each of the compound test wells were then divided by the averaged control values after background subtraction and multiplied by 100% to determine percent activity. EC₅₀ values for a protease inhibitor titration were calculated as the concentration which caused a 50% reduction in FRET or luciferase activity. The two numbers generated for the compound plate, percent cytoxicity and percent activity were used to determine compounds of interest for further analysis.

### HCV Replicon Luciferase Reporter Assay

The HCV replicon luciferase assay was developed to monitor the inhibitory effects of compounds described in the disclosure on HCV viral replication. Utilization of a replicon luciferase reporter assay was first described by Krieger et al (Krieger N, Lohmann V, and Bartenschlager R, J. Virol. 75(10):4614-4624 (2001)). HUH-7 cells, constitutively expressing the HCV replicon, were grown in Dulbecco's Modified Eagle Media (DMEM) (Gibco-BRL) containing 10% Fetal calf serum (FCS) (Sigma) and 1 mg/ml G418 (Gibco-BRL). Compounds were serially diluted 3 folds in DMSO for a twenty-point titration and subsequently transferred to sterile 384-well tissue-culture treated plates (Coming cat # 3571). The plates were then seeded with 50 µl of cells at a density of 3.0 x 10³ cells/well in DMEM containing 4 % FCS (final DMSO concentration at 0.5 %). After 3 days incubation at 37°C, cells were analyzed for Renilla Luciferase activity using the EnduRen as substrate (Promega cat #E6485). The EnduRen substrate was diluted in DMEM and then added to the plates to a final concentration of 7.5 µM. The plates were incubated for 2 hrs at 37°C and then read immediately for 30 seconds with Viewlux Imager (PerkinElmer) using a luminescence program. To assess cytotoxicity of compounds, CC₅₀ values were generated by multiplexing the EnduRen-containing plates with Cell Titer-Blue (Promega, cat # G8082). 3 µl of Cell-Titer Blue was added to each well and incubated for 8 hrs at 37°C. The fluorescence signal from each well was read, with an excitation wavelength at 525/10 nm and an emission wavelength of 598/10 nm, using the Viewlux Imager.

Representative data for compounds are reported in Table 1.

| Structure | IC₅₀ | EC₅₀ |
|---|---|---|
| 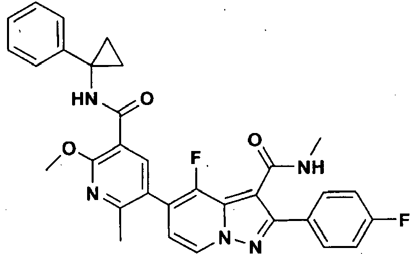 | | A |
| 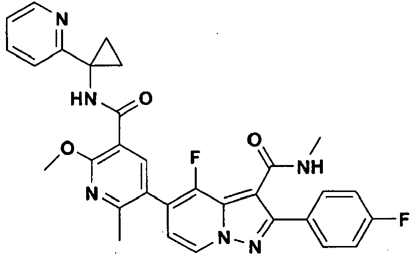 | | A |
| 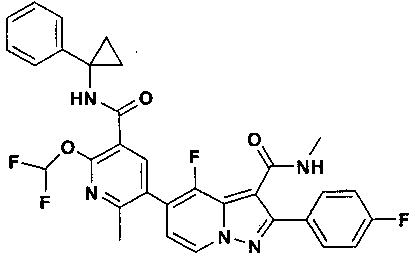 | | A |
| 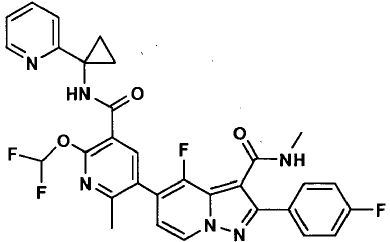 | | A |
| 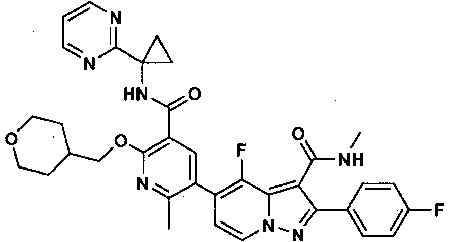 | | A |
| 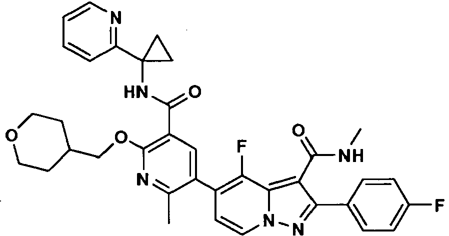 | | A |
| 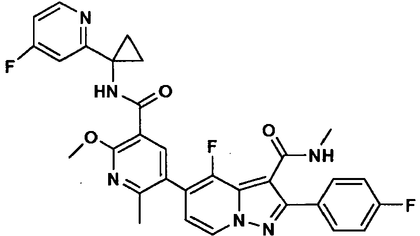 | | A |
| 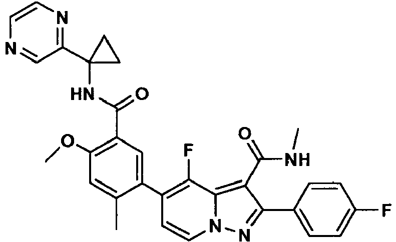 | | A |
| 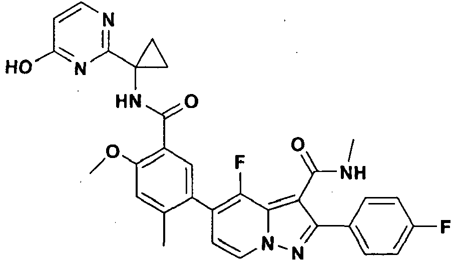 | | C |
| 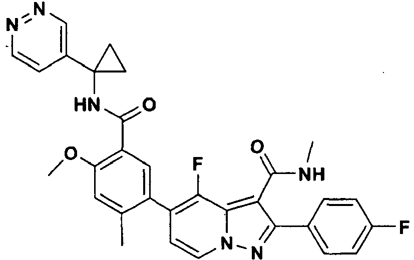 | | C |

A 0.002 to 0.25 µM; B >0.25 µM - < 1.0 µM; C 1.0 µM - 10.0 µM; D >0.67 µM but an exact value was not determined; E > 10.0 µM; F >0.4 µM; but an exact value was not determined; G > 1.39 µM but an exact value was not determined; H >0.62 µM but an exact value was not determined; I >4 µM but an exact value was not determined; J>3.7 µM but an exact value was not determined; K >1.23 µM but an exact value was not determined; L >4.17 µM but an exact value was not determined; M > 0.5 µM but an exact value was not determined.*EC50 determined using the 384 plate protocol and a 1a replicon.

### Pharmaceutical Compositions and Methods of Treatment

The compounds demonstrate activity against HCV NS5B and can be useful in treating HCV and HCV infection. Therefore, another aspect of the invention is a composition comprising a compound, or a pharmaceutically acceptable salt thereof, and a pharmaceutically acceptable carrier.

Another aspect of the invention is a composition further comprising a compound having anti-HCV activity.

Another aspect of the invention is a composition where the compound having anti-HCV activity is an interferon. Another aspect of the invention is where the interferon is selected from interferon alpha 2B, pegylated interferon alpha, consensus interferon, interferon alpha 2A, and lymphoblastoid interferon tau.

Another aspect of the invention is a composition where the compound having anti-HCV activity is a cyclosporin. Another aspect of the invention is where the cyclosporin is cyclosporin A.

Another aspect of the invention is a composition where the compound having anti-HCV activity is selected from the group consisting of interleukin 2, interleukin 6, interleukin 12, a compound that enhances the development of a type 1 helper T cell response, interfering RNA, anti-sense RNA, Imiqimod, ribavirin, an inosine 5'-monophospate dehydrogenase inhibitor, amantadine, and rimantadine.

Another aspect of the invention is a composition where the compound having anti-HCV activity is effective to inhibit the function of a target selected from HCV metalloprotease, HCV serine protease, HCV polymerase, HCV helicase, HCV NS4B protein, HCV entry, HCV assembly, HCV egress, HCV NS5A protein, IMPDH, and a nucleoside analog for the treatment of an HCV infection.

Another aspect of the invention is a composition comprising a compound, or a pharmaceutically acceptable salt thereof, a pharmaceutically acceptable carrier, an interferon and ribavirin.

Another aspect of the invention is a compound for use in a method of inhibiting the function of the HCV replicon comprising contacting the HCV replicon with a compound or a pharmaceutically acceptable salt thereof.

Another aspect of the invention is a compound for use in a method of inhibiting the function of the HCV NS5B protein comprising contacting the HCV NS5B protein with a compound or a pharmaceutically acceptable salt thereof.

Another aspect of the invention is a compound for use in a method of treating an HCV infection in a patient comprising administering to the patient a therapeutically effective amount of a compound or a pharmaceutically acceptable salt thereof. In another embodiment the compound is effective to inhibit the function of the HCV replicon. In another embodiment the compound is effective to inhibit the function of the HCV NS5B protein.

Another aspect of the invention is a compound for use in a method of treating an HCV infection in a patient comprising administering to the patient a therapeutically effective amount of a compound, or a pharmaceutically acceptable salt thereof, in conjunction with (prior to, after, or concurrently) another compound having anti-HCV activity.

Another aspect of the invention is the method where the other compound having anti-HCV activity is an interferon.

Another aspect of the invention is the method where the interferon is selected from interferon alpha 2B, pegylated interferon alpha, consensus interferon, interferon alpha 2A, and lymphoblastoid interferon tau.

Another aspect of the invention is the method where the other compound having anti-HCV activity is a cyclosporin.

Another aspect of the invention is the method where the cyclosporin is cyclosporin A.

Another aspect of the invention is the method where the other compound having anti-HCV activity is selected from interleukin 2, interleukin 6, interleukin 12, a compound that enhances the development of a type 1 helper T cell response, interfering RNA, anti-sense RNA, Imiqimod, ribavirin, an inosine 5'-monophospate dehydrogenase inhibitor, amantadine, and rimantadine.

Another aspect of the invention is the method where the other compound having anti-HCV activity is effective to inhibit the function of a target selected from the group consisting of HCV metalloprotease, HCV serine protease, HCV polymerase, HCV helicase, HCV NS4B protein, HCV entry, HCV assembly, HCV egress, HCV NS5A protein, IMPDH, and a nucleoside analog for the treatment of an HCV infection.

Another aspect of the invention is the method where the other compound having anti-HCV activity is effective to inhibit the function of target in the HCV life cycle other than the HCV NS5B protein.

"Therapeutically effective" means the amount of agent required to provide a meaningful patient benefit as understood by practitioners in the field of hepatitis and HCV infection.

"Patient" means a person infected with the HCV virus and suitable for therapy as understood by practitioners in the field of hepatitis and HCV infection.

"Treatment," "therapy," "regimen," "HCV infection," and related terms are used as understood by practitioners in the field of hepatitis and HCV infection.

The compounds of this invention are generally given as pharmaceutical compositions comprised of a therapeutically effective amount of a compound or its pharmaceutically acceptable salt and a pharmaceutically acceptable carrier and may contain conventional excipients. Pharmaceutically acceptable carriers are those conventionally known carriers having acceptable safety profiles. Compositions encompass all common solid and liquid forms including for example capsules, tablets, losenges, and powders as well as liquid suspensions, syrups, elixers, and solutions. Compositions are made using common formulation techniques, and conventional excipients (such as binding and wetting agents) and vehicles (such as water and alcohols) are generally used for compositions. See, for example, Remington's Pharmaceutical Sciences, Mack Publishing Company, Easton, PA, 17th edition, 1985.

Solid compositions are normally formulated in dosage units and compositions providing from about 1 to 1000 mg of the active ingredient per dose are preferred. Some examples of dosages are 1 mg, 10 mg, 100 mg, 250 mg, 500 mg, and 1000 mg. Generally, other agents will be present in a unit range similar to agents of that class used clinically. Typically, this is 0.25-1000 mg/unit.

Liquid compositions are usually in dosage unit ranges. Generally, the liquid composition will be in a unit dosage range of 1-100 mg/mL. Some examples of dosages are 1 mg/mL, 10 mg/mL, 25 mg/mL, 50 mg/mL, and 100 mg/mL. Generally, other agents will be present in a unit range similar to agents of that class used clinically. Typically, this is 1-100 mg/mL.

The invention encompasses all conventional modes of administration; oral and parenteral methods are preferred. Generally, the dosing regimen will be similar to other agents used clinically. Typically, the daily dose will be 1-100 mg/kg body weight daily. Generally, more compound is required orally and less parenterally. The specific dosing regime, however, will be determined by a physician using sound medical judgement.

The invention also encompasses methods where the compound is given in combination therapy. That is, the compound can be used in conjunction with, but separately from, other agents useful in treating hepatitis and HCV infection. In these combination methods, the compound will generally be given in a daily dose of 1-100 mg/kg body weight daily in conjunction with other agents. The other agents generally will be given in the amounts used therapeutically. The specific dosing regime, however, will be determined by a physician using sound medical judgement.

Some examples of compounds suitable for compositions and methods are listed in Table 2.

**Table 2.**

| Brand Name | Type of Inhibitor or Target | Source Company |
|---|---|---|
| Omega IFN | IFN-ω | Intarcia Therapeutics |
| BILN-2061 | serine protease inhibitor | Boehringer Ingelheim Pharma KG, Ingelheim, Germany |
| Summetrel | antiviral | Endo Pharmaceuticals Holdings Inc., Chadds Ford, PA |
| Roferon A | IFN-α2a | F. Hoffmann-La Roche LTD, Basel, Switzerland |
| Pegasys | PEGylated IFN-α2a | F. Hoffmann-La Roche LTD, Basel, Switzerland |
| Pegasys and Ribavirin | PEGylated IFN-α2a/ribavirin | F. Hoffmann-La Roche LTD, Basel, Switzerland |
| CellCept | HCV IgG immunosuppressant | F. Hoffmann-La Roche LTD, Basel, Switzerland |
| Wellferon | lymphoblastoid IFN-αn1 | GlaxoSmithKline plc, Uxbridge, UK |
| Albuferon - α | albumin IFN-α2b | Human Genome Sciences Inc., Rockville, MD |
| Levovirin | ribavirin | ICN Pharmaceuticals, Costa Mesa, CA |
| IDN-6556 | caspase inhibitor | Idun Pharmaceuticals Inc., San Diego, CA |
| IP-501 | antifibrotic | Indevus Pharmaceuticals Inc., Lexington, MA |
| Actimmune | INF-γ | InterMune Inc., Brisbane, CA |
| Infergen A | IFN alfacon-1 | InterMune Pharmaceuticals Inc., Brisbane, CA |
| ISIS 14803 | antisense | ISIS Pharmaceuticals Inc, Carlsbad, CA/Elan Phamaceuticals Inc., New York, NY |
| JTK-003 | RdRp inhibitor | Japan Tobacco Inc., Tokyo, Japan |
| Pegasys and Ceplene | PEGylated IFN-α2a/ immune modulator | Maxim Pharmaceuticals Inc., San Diego, CA |
| Ceplene | immune modulator | Maxim Pharmaceuticals Inc., San Diego, CA |
| Civacir | HCV IgG immunosuppressant | Nabi Biopharmaceuticals Inc., Boca Raton, FL |
| Intron A and Zadaxin | IFN-α2b/α1-thymosin | RegeneRx Biopharmiceuticals Inc., Bethesda, MD/ SciClone Pharmaceuticals Inc, San Mateo, CA |
| Levovirin | IMPDH inhibitor | Ribapharm Inc., Costa Mesa, CA |
| Viramidine | Ribavirin Prodrug | Ribapharm Inc., Costa Mesa, CA |
| Heptazyme | ribozyme | Ribozyme Pharmaceuticals Inc., Boulder, CO |
| Intron A | IFN-α2b | Schering-Plough Corporation, Kenilworth, NJ |
| PEG-Intron | PEGylated IFN-α2b | Schering-Plough Corporation, Kenilworth, NJ |
| Rebetron | IFN-α2b/ribavirin | Schering-Plough Corporation, Kenilworth, NJ |
| Ribavirin | ribavirin | Schering-Plough Corporation, Kenilworth, NJ |
| PEG-Intron / Ribavirin | PEGylated IFN-α2b/ribavirin | Schering-Plough Corporation, Kenilworth, NJ |
| Zadazim | Immune modulator | SciClone Pharmaceuticals Inc., San Mateo, CA |
| Rebif | IFN-β1a | Serono, Geneva, Switzerland |
| IFN-β and EMZ701 | IFN-β and EMZ701 | Transition Therapeutics Inc., Ontario, Canada |
| Batabulin (T67) | β-tubulin inhibitor | Tularik Inc., South San Francisco, CA |
| Merimepodib (VX-497) | IMPDH inhibitor | Vertex Pharmaceuticals Inc., Cambridge, MA |
| Telaprevir (VX-950, LY-570310) | NS3 serine protease inhibitor | Vertex Pharmaceuticals Inc., Cambridge, MA/ Eli Lilly and Co. Inc., Indianapolis, IN |
| Omniferon | natural IFN-α | Viragen Inc., Plantation, FL |
| XTL-6865 (XTL-002) | monoclonal antibody | XTL Biopharmaceuticals Ltd., Rehovot, Isreal |
| HCV-796 | NS5B Replicase Inhibitor | Wyeth / Viropharma |
| NM-283 | NS5B Replicase Inhibitor | Idenix / Novartis |
| GL-59728 | NS5B Replicase Inhibitor | Gene Labs / Novartis |
| GL-60667 | NS5B Replicase Inhibitor | Gene Labs / Novartis |
| 2'C MeA | NS5B Replicase Inhibitor | Gilead |
| PSI 6130 | NS5B Replicase Inhibitor | Roche |
| R1626 | NS5B Replicase Inhibitor | Roche |
| SCH 503034 | serine protease inhibitor | Schering Plough |
| NIM811 | Cyclophilin Inhibitor | Novartis |
| Suvus | Methylene blue | Bioenvision |
| Multiferon | Long lasting IFN | Viragen/Valentis |
| Actilon (CPG10101) | TLR9 agonist | Coley |
| Interferon-β | Interferon-β-1a | Serono |
| Zadaxin | Immunomodulator | Sciclone |
| Pyrazolopyrimidine compounds and salts From WO-2005047288 26 May 2005 | HCV Inhibitors | Arrow Therapeutics Ltd. |
| 2'C Methyl adenosine | NS5B Replicase Inhibitor | Merck |
| GS-9132 (ACH-806) | HCV Inhibitor | Achillion / Gilead |

### DESCRIPTION OF SPECIFIC EMBODIMENTS

Abbreviations used in the schemes generally follow conventions used in the art. Chemical abbreviations used in the specification and Examples are defined as follows: "NaHMDS" for sodium bis(trimethylsilyl)amide; "DMF" for N,N-dimethylformamide; "MeOH" for methanol; "NBS" for N-bromosuccinimide; "Ar" for aryl; "TFA" for trifluoroacetic acid; "LAH" for lithium aluminum hydride; "BOC", "DMSO" for dimethylsulfoxide; "h" for hours; "rt" for room temperature or retention time (context will dictate); "min" for minutes; "EtOAc" for ethyl acetate; "THF" for tetrahydrofuran; "EDTA" for ethylenediaminetetraacetic acid; "Et₂O" for diethyl ether; "DMAP" for 4-dimethylaminopyridine; "DCE" for 1,2-dichloroethane; "ACN" for acetonitrile; "DME" for 1,2-dimethoxyethane; "HOBt" for 1-hydroxybenzotriazole hydrate; "DIEA" for diisopropylethylamine, "Nf" for CF₃(CF₂)₃SO₂-; and "TMOF" for trimethylorthoformate.

Abbreviations as used herein, are defined as follows: "1 x" for once, "2 x" for twice, "3 x" for thrice, "°C" for degrees Celsius, "eq" for equivalent or equivalents, "g" for gram or grams, "mg" for milligram or milligrams, "L" for liter or liters, "mL" for milliliter or milliliters, "µL" for microliter or microliters, "N" for normal, "M" for molar, "mmol" for millimole or millimoles, "min" for minute or minutes, "h" for hour or hours, "rt" for room temperature, "RT" for retention time, "atm" for atmosphere, "psi" for pounds per square inch, "conc." for concentrate, "sat" or "sat'd " for saturated, "MW" for molecular weight, "mp" for melting point, "ee" for enantiomeric excess, "MS" or "Mass Spec" for mass spectrometry, "ESI" for electrospray ionization mass spectroscopy, "HR" for high resolution, "HRMS" for high resolution mass spectrometry , "LCMS" for liquid chromatography mass spectrometry, "HPLC" for high pressure liquid chromatography, "RP HPLC" for reverse phase HPLC, "TLC" or "tlc" for thin layer chromatography, "NMR" for nuclear magnetic resonance spectroscopy, "¹H" for proton, "δ" for delta, "s" for singlet, "d" for doublet, "t" for triplet, "q" for quartet, "m" for multiplet, "br" for broad, "Hz" for hertz, and "α", "β", "R", "S", "E", and "Z" are stereochemical designations familiar to one skilled in the art.

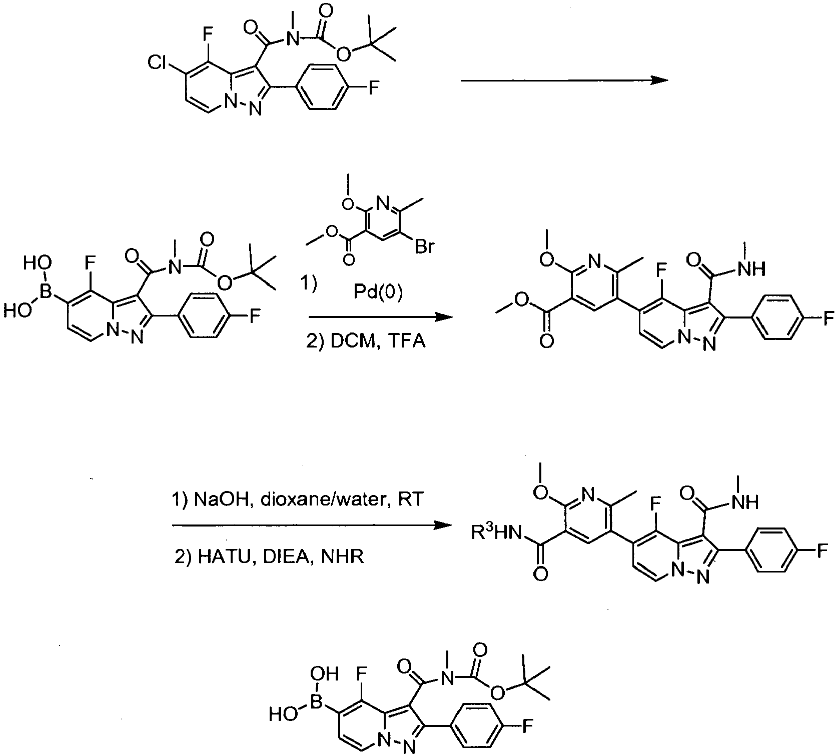

**3-(tert-butoxycarbonyl(methyl)carbamoyl)-4-fluoro-2-(4-fluorophenyl)pyrazolo[1,5-a]pyridin-5-ylboronic acid.** 4,4,4',4',5,5,5',5'-octamethyl-2,2'-bi(1,3,2-dioxaborolane) (0.45 g, 1.80 mmol), tert-butyl 5-chloro-4-fluoro-2-(4-fluorophenyl)pyrazolo[1,5-a]pyridine-3-carbonyl(methyl)carbamate (0.50 g, 1.18 mmol), potassium acetate (0.35 g, 3.56 mmol), 2-Dicyclohexylphosphino-2',4',6'-tri-iso-propyl-1,1'-biphenyl (0.011 g, 0.024 mmol) and Tris(dibenzylideneacetone)dipalladium (0) (0.011 g, 0.012 mmol) were combined in a round bottom flask and vacuum purged with nitrogen gas. Dioxane was added (2.4 mL) and the mixture was stirred at 70°C for 3h. The mixture was cooled to room temperature, filtered to remove solids and concentrated to a residue. The residue was purified using preparative HPLC (Waters- Xbridge, 50 x 100 mm, 5 micron, C18 column; 0.1M TFA, 0-100% B (B = 10% H₂O/CH₃OH)/ A (A = 90% H₂O/ CH₃OH), 12 min. gradient) to afford 3-(tert-butoxycarbonyl(methyl)carbamoyl)-4-fluoro-2-(4-fluorophenyl)pyrazolo[1,5-a]pyridin-5-ylboronic acid as a white solid. LCMS retention time: 2.820 min. LC data was recorded on a Shimadzu LC-10AS liquid chromatograph equipped with a Phenomenex-Luna, 3 micron, C18, 2.0 x 50 mm column using a SPD-10AV UV-Vis detector at a detector wave length of 220 nM. The elution conditions employed a flow rate of 0.8 mL/min, a gradient of 100% solvent A / 0% solvent B to 0% solvent A / 100% solvent B, a gradient time of 4 min, a hold time of 1 min, and an analysis time of 5 min where solvent A was 10% CH₃CN / 90% H₂O / 10 mM TFA and solvent B was 10% H₂O / 90% CH₃CN / 10 mM TFA. MS data was determined using a Micromass Platform for LC in electrospray mode. m/z 432 (MH⁺).

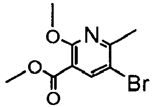

**Methyl 5-bromo-2-methoxy-6-methylnicotinate.** Bromine (3.59 mL, 69.7 mmol) was added dropwise to a cooled solution (0°C, ice bath) containing sodium hydroxide (15.6 g, 390 mmol) and water (120 mL). The solution was allowed to proceed at 0°C for 15 minutes and allowed to warm to room temperature. 2-hydroxy-6-methylnicotinic acid was added and the resulting solution was maintained for 20 minutes. The solution was again cooled (0°C, ice bath) and acidified to pH 1-2 by the dropwise addition of concentrated hydrochloric acid (20 mL). 5-bromo-2-hydroxy-6-methylnicotinic acid precipitated as a white solid and was collected by filtration. It was washed with water (500 mL) and allowed to air dry. m/z 233 (MH⁺).

To a solution containing the 5-bromo-2-hydroxy-6-methylnicotinic acid thus obtained (5.0 g, 21.5 mmol), methyliodide (8.08 mL, 129 mmol) and chloroform (431 mL) was added silver carbonate (29.7 g, 108 mmol). The mixture was quickly stirred in the dark for 4 days, filtered through celite and concentrated to a residue. Purification of the residue on silica gel (25-100% ethyl acetate/hexanes, 60 min gradient, 240g column) afforded methyl 5-bromo-2-methoxy-6-methylnicotinate as a white solid. 1H NMR (400 MHz, MeOD) δ ppm 8.23 (1 H, s), 3.97 (3 H, m), 3.87 (3 H, s), 2.58 (3 H, s). LCMS retention time: 2.382 min. LC data was recorded on a Shimadzu LC-10AS liquid chromatograph equipped with a Phenomenex-Luna, 10 micron, C18, 3.0 x 50 mm column using a SPD-10AV UV-Vis detector at a detector wave length of 220 nM. The elution conditions employed a flow rate of 4 mL/min, a gradient of 100% solvent A / 0% solvent B to 0% solvent A / 100% solvent B, a gradient time of 3 min, a hold time of 1 min, and an analysis time of 4 min where solvent A was 10% methanol / 90% H₂O / 10 mM TFA and solvent B was 10% H₂O / 90% methanol/ 10 mM TFA. MS data was determined using a Micromass Platform for LC in electrospray mode. m/z 261 (MH⁺).

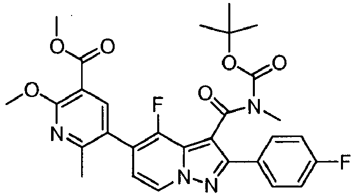

**Methyl 5-(3-(tert-butoxycarbonyl(methyl)carbamoyl)-4-fluoro-2-(4-fluorophenyl)pyrazolo[1,5-a]pyridin-5-yl)-2-methoxy-6-methylnicotinate**. To a degassed solution containing palladium (II) acetate (0.014 g, 0.063 mmol) and DMF (0.70 mL) was added 1,1' - Bis (diphenylphosphino)ferrocene. The solution was heated to 50°C and maintained for 15 minutes. The solution was cooled to room temperature and 3 -(tert-butoxycarbonyl(methyl)carbamoyl)-4-fluoro-2-(4-fluorophenyl)pyrazolo[1,5-a]pyridin-5-ylboronic acid (0.10 g, 0.21 mmol) and methyl 5-bromo-2-methoxy-6-methylnicotinate (0.054 g, 0.21 mmol) were added. Triethylamine was then added and the solution was heated at 90°C for 3 h. The mixture was cooled to room temperature, filtered to remove solids and concentrated. Purification on silica gel (0-75% ethyl acetate/hexanes, 60 min gradient, 25g column) afforded methyl 5-(3-(tert-butoxycarbonyl(methyl)carbamoyl)-4-fluoro-2-(4-fluorophenyl)pyrazolo[1,5-a]pyridin-5-yl)-2-methoxy-6-methylnicotinate as a white residue. 1H NMR (400 MHz, MeOD) δ ppm 8.60 (1 H, d, *J*=7.03 Hz), 8.13 (1 H, s), 7.77 (2 H, d, *J*=3.26 Hz), 7.75 (1 H, s), 7.24 (2 H, s), 6.99 (1 H, s), 4.07 (3 H, s), 3.89 (3 H, s), 3.29 (3 H, s), 2.45 (3 H, s), 1.01 - 1.13 (9 H, m). LCMS retention time: 3.803 min. LC data was recorded on a Shimadzu LC-10AS liquid chromatograph equipped with a Phenomenex-Luna, 3 micron, C18, 2.0 x 50 mm column using a SPD-10AV UV-Vis detector at a detector wave length of 220 nM. The elution conditions employed a flow rate of 0.8 mL/min, a gradient of 100% solvent A / 0% solvent B to 0% solvent A / 100% solvent B, a gradient time of 4 min, a hold time of 1 min, and an analysis time of 5 min where solvent A was 10% CH₃CN / 90% H₂O / 10 mM TFA and solvent B was 10% H₂O / 90% CH₃CN / 10 mM TFA. MS data was determined using a Micromass Platform for LC in electrospray mode. m/z 567 (MH⁺).

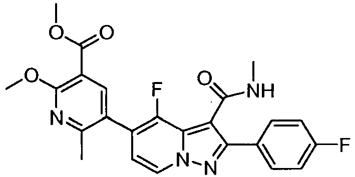

**Methyl 5-(4-fluoro-2-(4-fluorophenyl)-3-(methylcarbamoyl)pyrazolo[1,5-a]pyridin-5-yl)-2-methoxy-6-methylnicotinate**. To a solution containing methyl 5-(3-(tert-butoxycarbonyl(methyl)carbamoyl)-4-fluoro-2-(4-fluorophenyl)pyrazolo[1,5-a]pyridin-5-yl)-2-methoxy-6-methylnicotinate (0.07 g, 0.13 mmol) and dichloromethane (2.5 mL) was added TFA (0.39 mL, 5.0 mmol) at room temperature. The solution was maintained for 15 minutes and concentrated to dryness to afford Methyl 5-(4-fluoro-2-(4-fluorophenyl)-3-(methylcarbamoyl)pyrazolo[1,5-a]pyridin-5-yl)-2-methoxy-6-methylnicotinate as a tan solid which was used without further purification. LCMS retention time: 1.777 min. LC data was recorded on a Shimadzu LC-10AS liquid chromatograph equipped with a Phenomenex-Luna, 10 micron, C18, 3.0 x 50 mm column using a SPD-10AV UV-Vis detector at a detector wave length of 220 nM. The elution conditions employed a flow rate of 4 mL/min, a gradient of 100% solvent A / 0% solvent B to 0% solvent A / 100% solvent B, a gradient time of 3 min, a hold time of 1 min, and an analysis time of 4 min where solvent A was 10% methanol / 90% H₂O / 10 mM TFA and solvent B was 10% H₂O / 90% methanol/ 10 mM TFA. MS data was determined using a Micromass Platform for LC in electrospray mode. m/z 489 (MNa⁺). 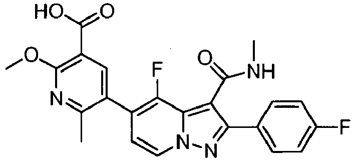 **5-(4-fluoro-2-(4-fluorophenyl)-3-(methylcarbamoyl)pyrazolo[1,5-a]pyridin-5-yl)-2-methoxy-6-methylnicotinic acid.** To a solution containing Methyl 5-(4-fluoro-2-(4-fluorophenyl)-3-(methylcarbamoyl)pyrazolo[1,5-a]pyridin-5-yl)-2-methoxy-6-methylnicotinate (0.041 g, 0.088 mmol) and dioxane (0.44 mL) was added aqueous sodium hydroxide (0.44 mL, 2.0 M). The solution was maintained at room temperature for 18 h. The solution was adjusted to below pH 4 with aqueous HCl (1.0 mL, 1.0 N). 5-(4-fluoro-2-(4-fluorophenyl)-3-(methylcarbamoyl)pyrazolo[1,5-a]pyridin-5-yl)-2-methoxy-6-methylnicotinic acid precipitated as a white solid and was filtered off, washed with water (3 x 2 mL) and air dried. 1H NMR (400 MHz, DMSO-*d*₆) δ ppm 12.98 (1 H, br. s.), 8.73 (1 H, d, *J*=7.03 Hz), 8.44 (1 H, q, *J*=4.52 Hz), 8.06 (1 H, s), 7.87 - 7.98 (2 H, m), 7.29 - 7.39 (2 H, m), 7.04 (1 H, t, *J*=7.03 Hz), 3.98 (3 H, s), 2.76 (3 H, d, *J*=4.52 Hz), 2.42 (3 H, s). LCMS retention time: 1.798 min. LC data was recorded on a Shimadzu LC-10AS liquid chromatograph equipped with a Phenomenex-Luna, 3 micron, C18, 2.0 x 50 mm column using a SPD-10AV UV-Vis detector at a detector wave length of 220 nM. The elution conditions employed a flow rate of 0.8 mL/min, a gradient of 100% solvent A / 0% solvent B to 0% solvent A / 100% solvent B, a gradient time of 4 min, a hold time of 1 min, and an analysis time of 5 min where solvent A was 5% CH₃CN / 95% H₂O / 10 mM ammonium acetate and solvent B was 5% H₂O / 95% CH₃CN / 10 mM ammonium acetate. MS data was determined using a Micromass Platform for LC in electrospray mode. m/z 453 (MH⁺).

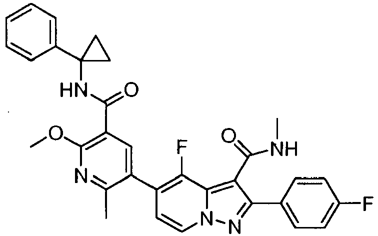

**4-fluoro-2-(4-fluorophenyl)-5-(6-methoxy-2-methyl-5-(1-phenylcyclopropylcarbamoyl)pyridin-3-yl)-N-methylpyrazolo[1,5-a]pyridine-3-carboxamide.** To a solution containing 1-phenylcyclopropanamine hydrochloride (0.0067 g, 0.040 mmol), diisopropylethylamine (0.14 mL, 0.79 mmol), 5-(4-fluoro-2-(4-fluorophenyl)-3-(methylcarbamoyl)pyrazolo[1,5-a]pyridin-5-yl)-2-methoxy-6-methylnicotinic acid (0.018 g, 0.040 mmol) and DMF (0.27 mL) was added HATU (0.030 g, 0.080 mmol) in one portion. The solution was maintained at room temperature for 1h and concentrated. Purification of the resultant residue using preparative HPLC (Waters- Xbridge, 50 x 100 mm, 5 micron, C18 column; 0.1M ammonium acetate, 0-100% B (B = 5% H₂O/CH₃CN)/ A (A = 95% H₂O/ CH₃CN), 15 min. gradient) afforded 4-fluoro-2-(4-fluorophenyl)-5-(6-methoxy-2-methyl-5-(1-phenylcyclopropylcarbamoyl)pyridin-3-yl)-N-methylpyrazolo[1,5-a]pyridine-3-carboxamide as a white solid. Preparative HPLC retention time: 13.94 min. 1H NMR (400 MHz, MeOD) δ ppm 8.54 (1 H, d, *J*=7.03 Hz), 8.16 (1 H, s), 7.84 - 7.95 (2 H, m), 7.29 (4 H, d, *J*=4.27 Hz), 7.14 - 7.26 (3 H, m), 6.93 (1 H, t, *J*=6.90 Hz), 4.18 (3 H, s), 2.85 - 2.96 (3 H, m), 2.48 (3 H, s), 1.37 (4 H, d, *J*=6.27 Hz). LCMS: retention time: 1.847 min. LC data was recorded on a Shimadzu LC-10AS liquid chromatograph equipped with a Phenomenex-Luna, 10u, C 18, 3.0 x 50 mm column using a SPD-10AV UV-Vis detector at a detector wave length of 220 nM. The elution conditions employed a flow rate of 4 mL/min, a gradient of 100% solvent A / 0% solvent B to 0% solvent A / 100% solvent B, a gradient time of 2 min, a hold time of 1 min, and an analysis time of 3 min where solvent A was 5% CH₃OH / 95% H₂O / 10 mM ammonium acetate and solvent B was 5% H₂O / 95% CH₃OH / 10 mM ammonium acetate. MS data was determined using a Micromass Platform for LC in electrospray mode. m/z 568 (MH⁺).

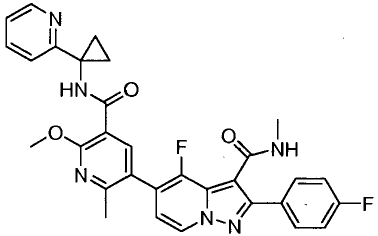

**4-fluoro-2-(4-fluorophenyl)-5-(6-methoxy-2-methyl-5-(1-(pyridin-2-yl)cyclopropylcarbamoyl)pyridin-3-yl)-N-methylpyrazolo[1,5-a]pyridine-3-carboxamide.** To a solution containing 1-(pyridin-2-yl)cyclopropanamine hydrochoride (0.0078 g, 0.046 mmol), diisopropylethylamine (0.049 mL, 0.28 mmol), 5-(4-fluoro-2-(4-fluorophenyl)-3-(methylcarbamoyl)pyrazolo[1,5-a]pyridin-5-yl)-2-methoxy-6-methylnicotinic acid (0.016 g, 0.035 mmol) and DMF (0.24 mL) was added HATU (0.027 g, 0.071 mmol) in one portion. The solution was maintained at room temperature for 1h and concentrated. Purification of the resultant residue using preparative HPLC (Waters- Xbridge, 50 x 100 mm, 5 micron, C18 column; 0.1M ammonium acetate, 0-100% B (B = 5% H₂O/CH₃CN)/ A (A = 95% H₂O/ CH₃CN), 15 min. gradient) afforded 4-fluoro-2-(4-fluorophenyl)-5-(6-methoxy-2-methyl-5-(1-(pyridin-2-yl)cyclopropylcarbamoyl)pyridin-3-yl)-N-methylpyrazolo[1,5-a]pyridine-3-carboxamide as a white solid. Preparative HPLC retention time: 12.49 min. 1H NMR (400 MHz, MeOD) δ ppm 8.55 (1 H, d, *J*=7.03 Hz), 8.41 - 8.46 (1 H, m), 8.20 (1 H, s), 7.82 - 7.93 (2 H, m), 7.72 (1 H, td, *J*=7.78, 1.76 Hz), 7.52 (1 H, d, *J*=8.03 Hz), 7.15 - 7.27 (3 H, m), 6.95 (1 H, t, *J*=6.90 Hz), 4.19 (3 H, s), 2.91 (3 H, s), 2.49 (3 H, d, *J*=1.00 Hz), 1.65 - 1.73 (2 H, m), 1.36 - 1.42 (2 H, m). LCMS: retention time: 1.695 min. LC data was recorded on a Shimadzu LC-10AS liquid chromatograph equipped with a Phenomenex-Luna, 10u, C18, 3.0 x 50 mm column using a SPD-10AV UV-Vis detector at a detector wave length of 220 nM. The elution conditions employed a flow rate of 4 mL/min, a gradient of 100% solvent A / 0% solvent B to 0% solvent A / 100% solvent B, a gradient time of 2 min, a hold time of 1 min, and an analysis time of 3 min where solvent A was 5% CH₃OH / 95% H₂O / 10 mM ammonium acetate and solvent B was 5% H₂O / 95% CH₃OH / 10 mM ammonium acetate. MS data was determined using a Micromass Platform for LC in electrospray mode. m/z 569 (MH⁺).

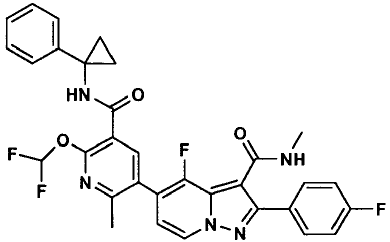

**5-(6-(difluoromethoxy)-2-methyl-5-(1-phenylcyclopropylcarbamoyl)pyridin-3-yl)-4-fluoro-2-(4-fluorophenyl)-N-methylpyrazolo[1,5-a]pyridine-3-carboxamide.** To a solution containing 1-phenylcyclopropanamine hydrochloride (0.0067 g, 0.040 mmol), diisopropylethylamine (0.43 mL, 0.25 mmol), 2-(difluoromethoxy)-5-(4-fluoro-2-(4-fluorophenyl)-3-(methylcarbamoyl)pyrazolo[1,5-a]pyridin-5-yl)-6-methylnicotinic acid (0.015 g, 0.031 mmol) and DMF (0.21 mL) was added HATU (0.023 g, 0.061 mmol) in one portion. The solution was maintained at room temperature for 1h and concentrated. Purification of the resultant residue using preparative HPLC (Waters- Xbridge, 50 x 100 mm, 5 micron, C18 column; 0.1M ammonium acetate, 0-100% B (B = 5% H₂O/CH₃CN)/ A (A = 95% H₂O/ CH₃CN), 15 min. gradient) afforded 5-(6-(difluoromethoxy)-2-methyl-5-(1-phenylcyclopropylcarbamoyl)pyridin-3-yl)-4-fluoro-2-(4-fluorophenyl)-N-methylpyrazolo[1,5-a]pyridine-3-carboxamide as a white solid. Preparative HPLC retention time: 11.1 min. 1H NMR (400 MHz, CHLOROFORM-*d*) δ ppm 8.46 - 8.49 (1 H, m), 8.39 (1 H, d, *J*=7.03 Hz), 7.98 (1 H, s), 7.78 - 7.89 (2 H, m), 7.73 (1 H, s), 7.29 - 7.39 (5 H, m), 7.14 - 7.25 (3 H, m), 6.71 (1 H, t, *J*=6.78 Hz), 5.80 (1 H, d, *J*=4.52 Hz), 2.98 (3 H, d, *J*=4.77 Hz), 2.49 (3 H, d, *J*=1.25 Hz), 1.41 (4 H, d, *J*=1.00 Hz). LCMS: retention time: 1.823 min. LC data was recorded on a Shimadzu LC-10AS liquid chromatograph equipped with a Phenomenex-Luna, 10 micron, C18, 3.0 x 50 mm column using a SPD-10AV UV-Vis detector at a detector wave length of 220 nM. The elution conditions employed a flow rate of 4 mL/min, a gradient of 100% solvent A / 0% solvent B to 0% solvent A / 100% solvent B, a gradient time of 2 min, a hold time of 1 min, and an analysis time of 3 min where solvent A was 5% CH₃OH / 95% H₂O / 10 mM ammonium acetate and solvent B was 5% H₂O / 95% CH₃OH / 10 mM ammonium acetate. MS data was determined using a Micromass Platform for LC in electrospray mode. m/z 604 (MH⁺).

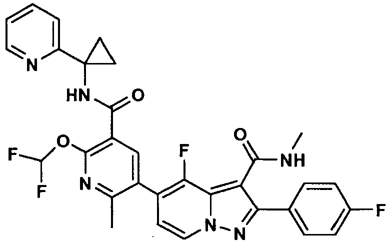

**5-(6-(difluoromethoxy)-2-methyl-5-(1-(pyridin-2-yl)cyclopropylcarbamoyl)pyridin-3-yl)-4-fluoro-2-(4-fluorophenyl)-N-methylpyrazolo[1,5-a]pyridine-3-carboxamide**. To a solution containing 1-(pyridin-2-yl)cyclopropanamine dihydrochloride (0.0082 g, 0.040 mmol), diisopropylethylamine (0.43 mL, 0.25 mmol), 2-(difluoromethoxy)-5-(4-fluoro-2-(4-fluorophenyl)-3-(methylcarbamoyl)pyrazolo[1,5-a]pyridin-5-yl)-6-methylnicotinic acid (0.01 g, 0.031 mmol) and DMF (0.21 mL) was added HATU (0.023 g, 0.061 mmol) in one portion. The solution was maintained at room temperature for 1h and concentrated. Purification of the resultant residue using preparative HPLC (Waters-Xbridge, 50 x 100 mm, 5 micron, C18 column; 0.1M ammonium acetate, 0-100% B (B = 5% H₂O/CH₃CN)/ A (A = 95% H₂O/ CH₃CN), 15 min. gradient) afforded 5-(6-(difluoromethoxy)-2-methyl-5-(1-(pyridin-2-yl)cyclopropylcarbamoyl)pyridin-3-yl)-4-fluoro-2-(4-fluorophenyl)-N-methylpyrazolo[1,5-a]pyridine-3-carboxamide as a white solid. Preparative HPLC retention time: 10.2 min. 1H NMR (400 MHz, CHLOROFORM-*d*) δ ppm 8.47 - 8.56 (2 H, m), 8.40 (1 H, d, *J*=7.03 Hz), 8.09 (1 H, br. s.), 7.80 - 7.90 (2 H, m), 7.75 (1 H, s), 7.57 (1 H, s), 7.42 (1 H, d, *J*=8.03 Hz), 7.09 - 7.22 (3 H, m), 6.73 (1 H, t, *J*=6.78 Hz), 5.80 (1 H, d, *J*=4.52 Hz), 2.98 (3 H, d, *J*=5.02 Hz), 2.51 (3 H, d, *J*=1.25 Hz), 1.77 - 1.86 (2 H, m), 1.39 - 1.50 (2 H, m). LCMS: retention time: 2.288 min. LC data was recorded on a Shimadzu LC-10AS liquid chromatograph equipped with a Phenomenex-Luna, 10 micron, C18, 3.0 x 50 mm column using a SPD-10AV UV-Vis detector at a detector wave length of 220 nM. The elution conditions employed a flow rate of 4 mL/min, a gradient of 100% solvent A / 0% solvent B to 0% solvent A / 100% solvent B, a gradient time of 3 min, a hold time of 1 min, and an analysis time of 4 min where solvent A was 5% CH₃OH / 95% H₂O / 10 mM ammonium acetate and solvent B was 5% H₂O / 95% CH₃OH / 10 mM ammonium acetate. MS data was determined using a Micromass Platform for LC in electrospray mode. m/z 605 (MH⁺).

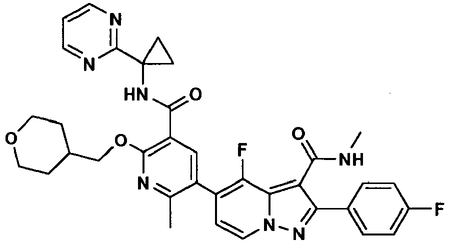

**4-fluoro-2-(4-fluorophenyl)-N-methyl-5-(2-methyl-5-(1-(pyrimidin-2-yl) cyclopropyl carbamoyl)-6-((tetrahydro-2H-pyran-4-yl)methoxy)pyridin-3-yl)pyrazolo[1,5-a]pyridine-3-carboxamide.** To a solution containing 1-(pyrimidin-2-yl)cyclopropanamine hydrochloride (0.0058 g, 0.034 mmol), diisopropylethylamine (0.36 mL, 0.21 mmol), 5-(4-fluoro-2-(4-fluorophenyl)-3-(methylcarbamoyl)pyrazolo[1,5-a]pyridin-5-yl)-6-methyl-2-((tetrahydro-2H-pyran-4-yl)methoxy)nicotinic acid (0.014 g, 0.026 mmol) and DMF (0.17 mL) was added HATU (0.020 g, 0.052 mmol) in one portion. The solution was maintained at room temperature for 1h and concentrated. Purification of the resultant residue using preparative HPLC (Waters- Xbridge, 50 x 100 mm, 5 micron, C18 column; 0.1M ammonium acetate, 0-100% B (B = 5% H₂O/CH₃CN)/ A (A = 95% H₂O/ CH₃CN), 15 min. gradient) afforded 4-fluoro-2-(4-fluorophenyl)-N-methyl-5-(2-methyl-5-(1-(pyrimidin-2-yl) cyclopropyl carbamoyl)-6-((tetrahydro-2H-pyran-4-yl)methoxy)pyridin-3-yl)pyrazolo[1,5-a]pyridine-3-carboxamide as a white solid. Preparative HPLC retention time: 10.6 min. 1H NMR (400 MHz, MeOD) δ ppm 8.65 (2 H, d, *J*=5.02 Hz), 8.55 (1 H, d, *J*=7.03 Hz), 8.25 (1 H, s), 7.83 - 7.93 (2 H, m), 7.18 - 7.28 (3 H, m), 6.95 (1 H, t, *J*=6.90 Hz), 4.52 (2 H, d, *J*=6.27 Hz), 3.99 (2 H, dd, *J*=11.29, 3.01 Hz), 3.48 (2 H, td, *J*=11.80, 2.01 Hz), 2.88 - 2.94 (3 H, m), 2.48 (3 H, d, *J*=1.00 Hz), 2.17 - 2.29 (1 H, m), 1.83 (1 H, d, *J*=1.76 Hz), 1.75 - 1.82 (3 H, m), 1.48 - 1.60 (4 H, m). LCMS: retention time: 2.333 min. LC data was recorded on a Shimadzu LC-10AS liquid chromatograph equipped with a Phenomenex-Luna, 10 micron, C18, 3.0 x 50 mm column using a SPD-10AV UV-Vis detector at a detector wave length of 220 nM. The elution conditions employed a flow rate of 4 mL/min, a gradient of 100% solvent A / 0% solvent B to 0% solvent A / 100% solvent B, a gradient time of 3 min, a hold time of 1 min, and an analysis time of 4 min where solvent A was 5% CH₃OH / 95% H₂O / 10 mM ammonium acetate and solvent B was 5% H₂O / 95% CH₃OH / 10 mM ammonium acetate. MS data was determined using a Micromass Platform for LC in electrospray mode. m/z 654 (MH⁺).

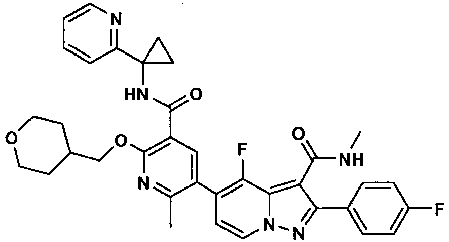

**4-fluoro-2-(4-fluorophenyl)-N-methyl-5-(2-methyl-5-(1-(pyridin-2-yl)cyclopropylcarbamoyl)-6-((tetrahydro-2H-pyran-4-yl)methoxy)pyridin-3-yl)pyrazolo[1,5-a]pyridine-3-carboxamide.** To a solution containing 1-(pyridin-2-yl)cyclopropanamine dihydrochloride (0.0054 g, 0.026 mmol), diisopropylethylamine (0.36 mL, 0.21 mmol), 5-(4-fluoro-2-(4-fluorophenyl)-3-(methylcarbamoyl)pyrazolo[1,5-a]pyridin-5-yl)-6-methyl-2-((tetrahydro-2H-pyran-4-yl)methoxy)nicotinic acid (0.014 g, 0.026 mmol) and DMF (0.17 mL) was added HATU (0.020 g, 0.052 mmol) in one portion. The solution was maintained at room temperature for 1h and concentrated. Purification of the resultant residue using preparative HPLC (Waters- Xbridge, 50 x 100 mm, 5 micron, C18 column; 0.1M ammonium acetate, 0-100% B (B = 5% H₂O/CH₃CN)/ A (A = 95% H₂O/ CH₃CN), 15 min. gradient) afforded 4-fluoro-2-(4-fluorophenyl)-N-methyl-5-(2-methyl-5-(1-(pyridin-2-yl)cyclopropylcarbamoyl)-6-((tetrahydro-2H-pyran-4-yl)methoxy)pyridin-3-yl)pyrazolo[1,5-a]pyridine-3-carboxamide as a white solid. Preparative HPLC retention time: 11.1 min. 1H NMR (400 MHz, MeOD) δ ppm 8.55 (1 H, d, *J*=7.03 Hz), 8.44 (1 H, d, *J*=4.02 Hz), 8.14 (1 H, s), 7.82 - 7.94 (2 H, m), 7.72 (1 H, td, *J*=7.65, 1.76 Hz), 7.53 (1 H, d, *J*=8.03 Hz), 7.15 - 7.28 (3 H, m), 6.94 (1 H, t, *J*=6.90 Hz), 4.50 (2 H, d, *J*=6.53 Hz), 4.00 (2 H, dd, *J=*11.29, 3.26 Hz), 3.48 (2 H, td, *J*=11.73, 1.88 Hz), 2.90 (3 H, s), 2.47 (3 H, s), 2.18 - 2.30 (1 H, m), 1.80 (2 H, dd, *J*=13.05, 1.76 Hz), 1.65 - 1.73 (2 H, m), 1.48 - 1.60 (2 H, m), 1.34 - 1.42 (2 H, m). LCMS: retention time: 2.332 min. LC data was recorded on a Shimadzu LC-10AS liquid chromatograph equipped with a Phenomenex-Luna, 10 micron, C18, 3.0 x 50 mm column using a SPD-10AV UV-Vis detector at a detector wave length of 220 nM. The elution conditions employed a flow rate of 4 mL/min, a gradient of 100% solvent A / 0% solvent B to 0% solvent A / 100% solvent B, a gradient time of 3 min, a hold time of 1 min, and an analysis time of 4 min where solvent A was 5% CH₃OH / 95% H₂O / 10 mM ammonium acetate and solvent B was 5% H₂O / 95% CH₃OH / 10 mM ammonium acetate. MS data was determined using a Micromass Platform for LC in electrospray mode. m/z 653 (MH⁺).

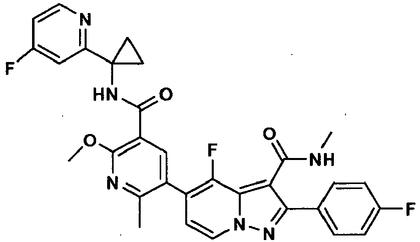

**4-fluoro-2-(4-fluorophenyl)-5-(5-(1-(4-fluoropyridin-2-yl)cyclopropylcarbamoyl)-6-methoxy-2-methylpyridin-3-yl)-N-methylpyrazolo[1,5-a]pyridine-3-carboxamide**. To a solution containing 1-(4-fluoropyridin-2-yl)cyclopropanamine (0.011 g, 0.044 mmol), diisopropylethylamine (0.62 mL, 0.35 mmol), 5-(4-fluoro-2-(4-fluorophenyl)-3-(methylcarbamoyl)pyrazolo[1,5-a]pyridin-5-yl)-2-methoxy-6-methylnicotinic acid (0.020 g, 0.044 mmol) and DMF (0.30 mL) was added HATU (0.034 g, 0.088 mmol) in one portion. The solution was maintained at room temperature for 15 minutes and concentrated. Purification of the resultant residue using preparative HPLC (Waters-Xbridge, 50 x 100 mm, 5 micron, C18 column; 0.1M ammonium acetate, 0-100% B (B = 5% H₂O/CH₃CN)/ A (A = 95% H₂O/ CH₃CN), 15 min. gradient) afforded 4-fluoro-2-(4-fluorophenyl)-5-(5-(1-(4-fluoropyridin-2-yl)cyclopropylcarbamoyl)-6-methoxy-2-methylpyridin-3-yl)-N-methylpyrazolo[1,5-a]pyridine-3-carboxamide as a white solid. Preparative HPLC retention time: 11.0 min. 1H NMR (400 MHz, MeOD) δ ppm 8.55 (1 H, d, *J*=7.03 Hz), 8.44 (1 H, dd, *J*=8.66, 5.65 Hz), 8.17 (1 H, s), 7.83 - 7.93 (2 H, m), 7.19 - 7.31 (3 H, m), 6.91 - 7.01 (2 H, m), 4.20 (3 H, s), 2.91 (3 H, s), 2.49 (3 H, d, *J*=1.00 Hz), 1.69 - 1.77 (2 H, m), 1.38 - 1.46 (2 H, m). LCMS: retention time: 2.018 min. LC data was recorded on a Shimadzu LC-10AS liquid chromatograph equipped with a Phenomenex-Luna, 3 micron, C 18, 2.0 x 30 mm column using a SPD-10AV UV-Vis detector at a detector wave length of 220 nM. The elution conditions employed a flow rate of 4 mL/min, a gradient of 100% solvent A / 0% solvent B to 0% solvent A / 100% solvent B, a gradient time of 2 min, a hold time of 1 min, and an analysis time of 3 min where solvent A was 5% CH₃OH / 95% H₂O / 10 mM ammonium acetate and solvent B was 5% H₂O / 95% CH₃OH / 10 mM ammonium acetate. MS data was determined using a Micromass Platform for LC in electrospray mode. m/z 587 (MH⁺).

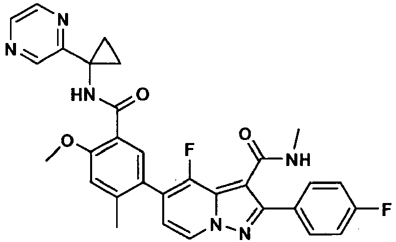

**4-fluoro-2-(4-fluorophenyl)-5-(4-methoxy-2-methyl-5-(1-(pyrazin-2-yl)cyclopropylcarbamoyl)phenyl)-N-methylpyrazolo[1,5-a]pyridine-3-carboxamide.** To a solution containing 1-(pyrazin-2-yl)cyclopropanamine 2,2,2-trifluoroacetate (0.014 g, 0.058 mmol), diisopropylethylamine (0.050 mL, 0.30 mmol), 5-(3-(tert-butoxycarbonyl(methyl)carbamoyl)-4-fluoro-2-(4-fluorophenyl)pyrazolo[1,5-a]pyridin-5-yl)-2-methoxy-4-methylbenzoic acid (0.020 g, 0.036 mmol) and DMF (0.24 mL) was added HATU (0.027 g, 0.073 mmol) in one portion. The solution was maintained at room temperature for 15 minutes. Dichloromethane (0.25 mL) was then added followed by trifluoroacetic acid (0.078 mL, 1.0 mmol). The solution was maintained for an additional 15 min and concentrated. The resultant residue was purified using preparative HPLC (Waters-Xbridge, 50 x 100 mm, 5 micron, C18 column; 0.1M ammonium acetate, 0-100% B (B = 5% H₂O/CH₃CN)/ A (A = 95% H₂O/ CH₃CN), 15 min. gradient) afforded 4-fluoro-2-(4-fluorophenyl)-5-(4-methoxy-2-methyl-5-(1-(pyrazin-2-yl)cyclopropylcarbamoyl)phenyl)-N-methylpyrazolo[1,5-a]pyridine-3-carboxamide as a white solid. Preparative HPLC retention time: 10.6 min. 1H NMR (400 MHz, MeOD) δ ppm 8.70 (1 H, d, *J=*1.51 Hz), 8.51 (1 H, s), 8.50 (1 H, dd, *J*=3.51, 1.76 Hz), 8.35 (1 H, d, *J*=2.51 Hz), 7.84 - 7.90 (2 H, m), 7.79 (1 H, s), 7.17 - 7.26 (3 H, m), 6.89 (1 H, t, *J*=6.90 Hz), 4.09 (3 H, s), 2.90 (3 H, s), 2.36 (3 H, s), 1.69 - 1.75 (2 H, m), 1.41 - 1.46 (2 H, m). LCMS: retention time: 1.942 min. LC data was recorded on a Shimadzu LC-10AS liquid chromatograph equipped with a Phenomenex-Luna, 3 micron, C18, 2.0 x 30 mm column using a SPD-10AV UV-Vis detector at a detector wave length of 220 nM. The elution conditions employed a flow rate of 4 mL/min, a gradient of 100% solvent A / 0% solvent B to 0% solvent A / 100% solvent B, a gradient time of 2 min, a hold time of 1 min, and an analysis time of 3 min where solvent A was 5% CH₃OH / 95% H₂O / 10 mM ammonium acetate and solvent B was 5% H₂O / 95% CH₃OH / 10 mM ammonium acetate. MS data was determined using a Micromass Platform for LC in electrospray mode. m/z 569 (MH⁺).

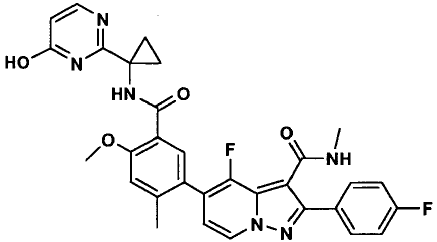

**4-fluoro-2-(4-fluorophenyl)-5-(5-(1-(4-hydroxypyrimidin-2-yl)cyclopropylcarbamoyl)-4-methoxy-2-methylphenyl)-N-methylpyrazolo[1,5-a]pyridine-3-carboxamide.** To a solution containing 2-(1-aminocyclopropyl)pyrimidin-4-ol hydrochloride (0.025 g, 0.13 mmol), diisopropylethylamine (0.15 mL, 0.89 mmol), 5-(3-(tert-butoxycarbonyl(methyl)carbamoyl)-4-fluoro-2-(4-fluorophenyl)pyrazolo[1,5-a]pyridin-5-yl)-2-methoxy-4-methylbenzoic acid (0.070 g, 0.11 mmol) and DMF (0.74 mL) was added HATU (0.085 g, 0.22 mmol) in one portion. The solution was maintained at room temperature for 15 minutes. Dichloromethane (0.22 mL) was then added followed by trifluoroacetic acid (0.068 mL, 0.88 mmol). The solution was maintained for an additional 15 min and concentrated. The resultant residue was purified using preparative HPLC (Waters- Xbridge, 50 x 100 mm, 5 micron, C18 column; 0.1M ammonium acetate, 0-100% B (B = 5% H₂O/CH₃CN)/ A (A = 95% H₂O/ CH₃CN), 15 min. gradient) afforded 4-fluoro-2-(4-fluorophenyl)-5-(5-(1-(4-hydroxypyrimidin-2-yl)cyclopropylcarbamoyl)-4-methoxy-2-methylphenyl)-N-methylpyrazolo[1,5-a]pyridine-3-carboxamide as a white solid. Preparative HPLC retention time: 9.4 min. 1H NMR (400 MHz, MeOD) δ ppm 8.50 (1 H, d, *J*=7.03 Hz), 7.78 - 7.90 (4 H, m), 7.17 - 7.29 (3 H, m), 6.87 (1 H, t, *J*=6.90 Hz), 6.22 (1 H, br. s.), 4.10 (3 H, s), 2.87 - 2.93 (3 H, m), 2.30 - 2.39 (3 H, m), 1.81 (2 H, br. s.), 1.40 - 1.48 (2 H, m). LCMS: retention time: 1.798 min. LC data was recorded on a Shimadzu LC-10AS liquid chromatograph equipped with a Phenomenex-Luna, 3 micron, C18, 2.0 x 30 mm column using a SPD-10AV UV-Vis detector at a detector wave length of 220 nM. The elution conditions employed a flow rate of 4 mL/min, a gradient of 100% solvent A / 0% solvent B to 0% solvent A / 100% solvent B, a gradient time of 2 min, a hold time of 1 min, and an analysis time of 3 min where solvent A was 5% CH₃OH / 95% H₂O / 10 mM ammonium acetate and solvent B was 5% H₂O / 95% CH₃OH / 10 mM ammonium acetate. MS data was determined using a Micromass Platform for LC in electrospray mode. m/z 585 (MH⁺).

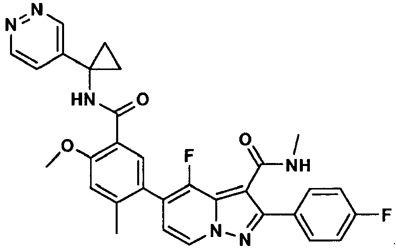

**4-fluoro-2-(4-fluorophenyl)-5-(4-methoxy-2-methyl-5-(1-(pyridazin-4-yl)cyclopropylcarbamoyl)phenyl)-N-methylpyrazolo[1,5-a]pyridine-3-**carboxamide. To a solution containing 1-(pyridazin-4-yl)cyclopropanamine trifluoroacetate. (0.018 g, 0.076 mmol), diisopropylethylamine (0.076 mL, 0.44 mmol), 5-(3-(tert-butoxycarbonyl(methyl)carbamoyl)-4-fluoro-2-(4-fluorophenyl)pyrazolo[1,5-a]pyridin-5-yl)-2-methoxy-4-methylbenzoic acid (0.030 g, 0.054 mmol) and DMF (0.37 mL) was added HATU (0.041 g, 0.11 mmol) in one portion. The solution was maintained at room temperature for 15 minutes. Dichloromethane (0.36 mL) was then added followed by trifluoroacetic acid (0.11 mL, 1.4 mmol). The solution was maintained for an additional 15 min and concentrated. The resultant residue was purified using preparative HPLC (Waters-Xbridge, 50 x 100 mm, 5 micron, C18 column; 0.1M ammonium acetate, 0-100% B (B = 5% H₂O/CH₃CN)/ A (A = 95% H₂O/ CH₃CN), 15 min. gradient) afforded 4-fluoro-2-(4-fluorophenyl)-5-(4-methoxy-2-methyl-5-(1-(pyridazin-4-yl)cyclopropylcarbamoyl)phenyl)-N-methylpyrazolo[1,5-a]pyridine-3-carboxamide as a white solid. Preparative HPLC retention time: 9.7 min. 1H NMR (400 MHz, MeOD) δ ppm 9.05 - 9.15 (3 H, m), 8.50 (1 H, d, *J*=7.03 Hz), 7.83 - 7.92 (2 H, m), 7.76 - 7.80 (1 H, m), 7.56 (1 H, dd, *J*=5.65, 2.64 Hz), 7.15 - 7.26 (3 H, m), 6.88 (1 H, t, *J*=6.78 Hz), 4.04 - 4.13 (3 H, m), 2.88 - 2.92 (3 H, m), 2.36 (3 H, s), 1.63 (5 H, s). LCMS: retention time: 1.798 min. LC data was recorded on a Shimadzu LC-10AS liquid chromatograph equipped with a Phenomenex-Luna, 3 micron, C18, 2.0 x 30 mm column using a SPD-10AV UV-Vis detector at a detector wave length of 220 nM. The elution conditions employed a flow rate of 4 mL/min, a gradient of 100% solvent A / 0% solvent B to 0% solvent A / 100% solvent B, a gradient time of 2 min, a hold time of 1 min, and an analysis time of 3 min where solvent A was 5% CH₃OH / 95% H₂O / 10 mM ammonium acetate and solvent B was 5% H₂O / 95% CH₃OH / 10 mM ammonium acetate. MS data was determined using a Micromass Platform for LC in electrospray mode. m/z 569 (MH⁺).

It will be evident to one skilled in the art that the present disclosure is not limited to the foregoing illustrative examples, and that it can be embodied in other specific forms without departing from the essential attributes thereof. It is therefore desired that the examples be considered in all respects as illustrative and not restrictive, reference being made to the appended claims, rather than to the foregoing examples.

## Claims

1. A compound selected from the group consisting of
4-fluoro-2-(4-fluorophenyl)-5-(6-methoxy-2-methyl-5-(1-phenylcyclopropylcarbamoyl)pyridin-3-yl)-N-methylpyrazolo[1,5-a]pyridine-3-carboxamide;
4-fluoro-2-(4-fluorophenyl)-5-(6-methoxy-2-methyl-5-(1-(pyridin-2-yl)cyclopropylcarbamoyl)pyridin-3-yl)-N-methylpyrazolo[1,5-a]pyridine-3-carboxamide;
5-((6-(difluoromethoxy)-2-methyl-5-(1-phenylcyclopropylcarbamoyl)pyridin-3-yl)-4-fluoro-2-(4-fluorophenyl)-N-methylpyrazolo[1,5-a]pyridine-3-carboxamide;
5-((6-(difluoromethoxy)-2-methyl-5-(1-(pyridin-2-yl)cyclopropylcarbamoyl)pyridin-3-yl)-4-fluoro-2-(4-fluorophenyl)-N-methylpyrazolo[1,5-a]pyridine-3-carboxamide;
4-fluoro-2-(4-fluorophenyl)-N-methyl-5-(2-methyl-5-(1-(pyrimidin-2-yl) cyclopropyl carbamoyl)-6-((tetrahydro-2H-pyran-4-yl)methoxy)pyridin-3-yl)pyrazolo[1,5-a]pyridine-3-carboxamide;
4-fluoro-2-(4-fluorophenyl)-N-methyl-5-(2-methyl-5-(1-(pyridin-2-yl)cyclopropylcarbamoyl)-6-((tetrahydro-2H-pyran-4-yl)methoxy)pyridin-3-yl)pyrazolo[1,5-a]pyridine-3-carboxamide;
4-fluoro-2-(4-fluorophenyl)-5-(5-(1-(4-fluoropyridin-2-yl)cyclopropylcarbamoyl)-6-methoxy-2-methylpyridin-3-yl)-N-methylpyrazolo[1,5-a]pyridine-3-carboxamide;
4-fluoro-2-(4-fluorophenyl)-5-(4-methoxy-2-methyl-5-(1-(pyrazin-2-yl)cyclopropylcarbamoyl)phenyl)-N-methylpyrazolo[1,5-a]pyridine-3-carboxamide;
4-fluoro-2-(4-fluorophenyl)-5-(5-(1-(4-hydroxypyrimidin-2-yl)cyclopropylcarbamoyl)-4-methoxy-2-methylphenyl)-N-methylpyrazolo[1,5-a]pyridine-3-carboxamide; and
4-fluoro-2-(4-fluorophenyl)-5-(4-methoxy-2-methyl-5-(1-(pyridazin-4-yl)cyclopropylcarbamoyl)phenyl)-N-methylpyrazolo[1,5-a]pyridine-3-carboxamide;
or a pharmaceutically acceptable salt thereof.

2. A composition comprising a compound of claim 1, or a pharmaceutically acceptable salt thereof, and a pharmaceutically acceptable carrier.

3. A compound of claim 1, or a pharmaceutically acceptable salt thereof for use in a method of treating hepatitis C infection.

## Patentansprüche

1. Verbindung, ausgewählt aus:
4-Fluor-2-(4-fluorphenyl)-5-(6-methoxy-2-methyl-5-(1-phenylcyclopropylcarbamoyl)pyridin-3-yl)-N-methylpyrazolo[1,5-α]pyridin-3-carboxamid;
4-Fluor-2-(4-fluorphenyl)-5-(6-methoxy-2-methyl-5-(1-(pyridin-2-yl)cyclopropylcarbamoyl)pyridin-3-yl)-N-methylpyrazolo[1,5-α]pyridin-3-carboxamid;
5-((6-(Difluormethoxy)-2-methyl-5-(1-phenylcyclopropylcarbamoyl)pyridin-3-yl)-4-fluor-2-(4-fluorphenyl)-N-methylpyrazolo[1,5-α]pyridin-3-carboxamid;
5-((6-(Difluormethoxy)-2-methyl-5-(1-(pyridin-2-yl)cyclopropylcarbamoyl)pyridin-3-yl)-4-fluor-2-(4-fluorphenyl)-N-methylpyrazolo[1,5-α]pyridin-3-carboxamid;
4-Fluor-2-(4-fluorphenyl)-N-methyl-5-(2-methyl-5-(1-(pyrimidin-2-yl)cyclopropylcarbamoyl)-6-((tetrahydro-2H-pyran-4-yl)methoxy)pyridin-3-yl)pyrazolo[1,5-α]pyridin-3-carboxamid;
4-Fluor-2-(4-fluorphenyl)-N-methyl-5-(2-methyl-5-(1-(pyridin-2-yl)cyclopropylcarbamoyl)-6-((tetrahydro-2H-pyran-4-yl)methoxy)pyridin-3-yl)pyrazolo[1,5-α]pyridin-3-carboxamid;
4-Fluor-2-(4-fluorphenyl)-5-(5-(1-(4-fluorpyridin-2-yl)cyclopropylcarbamoyl)-6-methoxy-2-methylpyridin-3-yl)-N-methylpyrazolo[1,5-α]pyridin-3-carboxamid;
4-Fluor-2-(4-fluorphenyl)-5-(4-methoxy-2-methyl-5-(1-(pyrazin-2-yl)cyclopropylcarbamoyl)phenyl)-N-methylpyrazolo[1,5-α]pyridin-3-carboxamid;
4-Fluor-2-(4-fluorphenyl)-5-(5-(1-(4-hydroxypyrimidin-2-yl)cyclopropylcarbamoyl)-4-methoxy-2-methylphenyl)-N-methylpyrazolo[1,5-α]pyridin-3-carboxamid; und
4-Fluor-2-(4-fluorphenyl)-5-(4-methoxy-2-methyl-5-(1-(pyridazin-4-yl)cyclopropylcarbamoyl)phenyl)-N-methylpyrazolo[1,5-α]pyridin-3-carboxamid;
oder ein pharmazeutisch verträgliches Salz davon.

2. Zusammensetzung, umfassend eine Verbindung nach Anspruch 1, oder ein pharmazeutisch verträgliches Salz davon und einen pharmazeutisch verträglichen Träger.

3. Verbindung nach Anspruch 1, oder ein pharmazeutisch verträgliches Salz davon zur Verwendung in einem Verfahren zur Behandlung einer Hepatitis-C-Infektion.

## Revendications

1. Composé sélectionné parmi le groupe constitué du
4-fluoro-2-(4-fluorophényl)-5-(6-méthoxy-2-méthyl-5-(1-phénylcyclopropylcarbamoyl)pyridin-3-yl)-N-méthylpyrazolo[1,5-a]pyridine-3-carboxamide;
4-fluoro-2-(4-fluorophényl)-5-(6-méthoxy-2-méthyl-5-(1-(pyridin-2-yl)cycloprapylcarbamoyl)pyridin-3-yl)-N-méthylpyrazolo[1,5-a]pyridine-3-carboxamide;
5-((6-(difluorométhoxy)-2-méthyl-5-(1-phénylcyclopropylcarbamoyl)pyridin-3-yl)-4-fluoro-2-(4-fluorophényl)-N-méthylpyrazolo[1,5-a]pyridine-3-carboxamide;
5-((6-(difluorométhoxy)-2-méthyl-5-(1-(pyridin-2-yl)cyclopropylcarbamoyl)pyridin-3-yl)-4-fluoro-2-(4-fluorophényl)-N-méthylpyrazolo[1,5-a]pyridine-3-carboxamide;
4-fluoro-2-(4-fluorophényl)-N-méthyl-5-(2-méthyl-5-(1-(pyrimidin-2-yl)cyclopropylcarbamoyl)-6-((tétrahydro-2H-pyran-4-yl)méthoxy)pyridin-3-yl)pyrazolo[1,5-a]pyridine-3-carboxamide;
4-fluoro-2-(4-fluorophényl)-N-méthyl-5-(2-méthyl-5-(1-(pyridin-2-yl)cyclopropylcarbamoyl)-6-((tétrahydro-2H-pyran-4-yl)méthoxy)pyridin-3-yl)pyrazolo[1,5-a]pyridine-3-carboxamide;
4-fluoro-2-(4-fluorophényl)-5-(5-(1-(4-fluoropyridin-2-yl)cyclopropylcarbamoyl)-6-méthoxy-2-méthylpyridin-3-yl)-N-methylpyrazolo[1,5-a]pyridine-3-carboxamide;
4-fluoro-2-(4-fluorophényl)-5-(4-méthoxy-2-méthyl-5-(1-(pyrazin-2-yl)cyclopropylcarbamoyl)phényl)-N-méthylpyrazolo[1,5-a]pyridine-3-carboxamide;
4-fluoro-2-(4-fluorophényl)-5-(5-(1-(4-hydroxypyrimidin-2-yl)cyclopropylcarbamoyl)-4-méthoxy-2-méthylphényl)-N-méthylpyrazolo[1,5-a]pyridine-3-carboxamide; et
4-fluoro-2-(4-fluorophényl)-5-(4-méthoxy-2-méthyl-5-(1-(pyridazin-4-yl)cyclopropylcarbamoyl)phényl)-N-méthylpyrazolo[1,5-a]pyridine-3-carboxamide;
ou sel pharmaceutiquement acceptable de celui-ci.

2. Composition comprenant un composé selon la revendication 1, ou sel pharmaceutiquement acceptable de celui-ci, et support pharmaceutiquement acceptable.

3. Composé selon la revendication 1, ou sel pharmaceutiquement acceptable de celui-ci pour l'utilisation dans un procédé de traitement de l'hépatite C.
